# EUROPEAN PATENT APPLICATION

(11) **EP 4 604 673 A1**
(43) Date of publication of application: **20.08.2025**
(21) Application number: 22963383.9
(22) Date of filing: 24.10.2022
(51) Int. Cl.: H05B 3/02, H05B 3/20, A24F 40/42, A24F 40/46

(54) **AEROSOL GENERATION SYSTEM**

(71) Applicant: Japan Tobacco Inc., Tokyo 105-6927 (JP)
(72) Inventor: YAMADA, Manabu, Tokyo 130-8603 (JP); INOUE, Yasunobu, Tokyo 130-8603 (JP)
(74) Representative: Hoffmann Eitle
(86) International application number: PCT/JP2022/039477
(87) International publication number: WO 2024/089731

(57) **Abstract**

[Problem] To provide a mechanism capable of further improving the quality of a user experience.

[Solution] An aerosol generation system comprising a tubular body that accommodates a substrate containing an aerosol source, a plurality of resistive heating layers that are laminated onto the outer side of a side wall of the tubular body, and a first heat diffusion layer that is laminated onto the outer side of the side wall of the tubular body, inward of the resistive heating layers.

## Description

### [TECHNICAL FIELD]

The present disclosure relates to an aerosol generation system.

### [BACKGROUND ART]

Inhalation devices that generate substances to be inhaled by a user, such as e-cigarettes and nebulizers, are in widespread use. For example, an inhalation device employs an aerosol source for generating an aerosol, and a substrate including a flavor source or the like for imparting a flavor component to the generated aerosol, to generate an aerosol to which the flavor component has been imparted. The user can enjoy the flavor by inhaling the aerosol to which the flavor component has been imparted, generated by the inhalation device. The action by which the user inhales the aerosol is also referred to below as "puffing" or a "puffing action".

There is a demand for improved heating efficiency in inhalation devices of a type in which an aerosol is generated by heating a substrate. For example, PTL1 listed below discloses a technique in which a coating of an electrically insulating material is formed on the surface of a heating chamber having an opening portion for accepting a substrate, and a coating of an electrically conductive material that acts as a Joule heater is additionally formed on the electrically insulating material.

### [CITATION LIST]

### [PATENT LITERATURE]

[PTL1] WO 2022/167261

### [SUMMARY OF INVENTION]

### [TECHNICAL PROBLEM]

However, the technique disclosed in PTL1 has only recently been developed, and there is still room for improvement in various aspects.

Accordingly, the present disclosure takes account of the abovementioned problems, and the objective of the present disclosure is to provide a mechanism capable of further improving the quality of the user experience.

### [SOLUTION TO PROBLEM]

In order to solve the above problem, one aspect of the present invention provides an aerosol generation system comprising a tubular body that accommodates a substrate containing an aerosol source, a plurality of resistive heating layers that are laminated onto the outer side of a side wall of the tubular body, and a first heat diffusion layer that is laminated onto the outer side of the side wall of the tubular body, inward of the resistive heating layers.

The side wall of the tubular body may include a plurality of first side walls having a planar outer surface and a plurality of second side walls different from the first side walls, wherein: the first side walls and the second side walls are arranged alternately along the circumferential direction of the tubular body; and two of the resistive heating layers are laminated using a vapor deposition process or a printing process onto the outer sides of two of the first side walls that are adjacent to and on both sides of the second side walls, in a state in which the resistive heating layers are spaced apart at the second side walls.

The aerosol generation system may further comprise a plurality of first electrically insulating layers that are laminated using a vapor deposition process or a printing process onto the outer sides of the first side walls, inward of the resistive heating layers, and the tubular body may be made of an electrically conductive material.

The part of the outer periphery of the tubular body on which the first electrically insulating layers are laminated may occupy less than 50% of the outer periphery of the tubular body.

The first electrically insulating layers may have a shape that conforms to the resistive heating layers.

The aerosol generation system may further comprise a plurality of second electrically insulating layers that are laminated outward of the resistive heating layers using a vapor deposition process or a printing process, and at least portions of the resistive heating layers may be sandwiched between the first electrically insulating layers and the second insulating layers.

The aerosol generation system may further comprise a power source unit for supplying power to the resistive heating layers, the first heat diffusion layer may be made of a material having a higher electrical conductivity than the tubular body, the first electrically insulating layers may be laminated outward of the first heat diffusion layer, and at least one of the two end portions of each resistive heating layer may protrude from the first electrically insulating layer and be connected to the first heat diffusion layer, and may be electrically connected to the power source unit via the first heat diffusion layer.

At least one of the two end portions of each resistive heating layer may protrude from the first electrically insulating layer and be connected to the first heat diffusion layer, and may be electrically connected via the first heat diffusion layer to another resistive heating layer adjacent to the resistive heating layer.

A conducting wire that is connected to the power source unit may be connected to the tubular body, and one of the two end portions of each resistive heating layer may protrude from the first electrically insulating layer and be connected to the first heat diffusion layer, and be electrically connected via the first heat diffusion layer and the tubular body to the conducting wire that is connected to the tubular body.

The end portion that protrudes from each first electrically insulating layer, among the two end portions of each resistive heating layer, may be connected to the first heat diffusion layer on the first side wall.

The end portion that protrudes from each first electrically insulating layer, among the two end portions of each resistive heating layer, may protrude from the first side wall and be connected to the first heat diffusion layer on the second side wall.

A conducting wire that is connected to the power source unit may be connected to one of the two end portions of the resistive heating layer.

The aerosol generation system may further comprise a power source unit for supplying power to the resistive heating layers, and a conducting wire that is connected to the power source unit may be connected to each of the two end portions of each resistive heating layer.

Among the two end portions of each resistive heating layer, the end portion to which the conducting wire that is connected to the power source unit is connected may be configured to be wider than other parts thereof.

The aerosol generation system may further comprise a second heat diffusion layer that is wrapped and laminated onto the outer side of the side wall of the tubular body, outward of the resistive heating layers.

The aerosol generation system may further comprise a heat insulating layer that is wrapped and laminated onto the outer side of the side wall of the tubular body, outward of the resistive heating layers.

The aerosol generation system may further comprise a power source unit for supplying power to the resistive heating layers, and at least one of the two end portions of each resistive heating layer may protrude from the first electrically insulating layer and be connected to the tubular body, and may be electrically connected to the power source unit via the tubular body.

The first side walls may be flat plates, the second side walls may be curved plates that are curved to the outside of the tubular body along the circumferential direction of the tubular body, and the substrate accommodated in the tubular body may be pressed by the first side walls.

The first side walls may be flat plates, the second side walls may be flat plates, the length of the first side walls in the circumferential direction of the tubular body may be greater than the length of the second side walls, and the substrate accommodated in the tubular body may be pressed by the first side walls.

The aerosol generation system may further comprise the substrate.

### [ADVANTAGEOUS EFFECTS OF INVENTION]

The present disclosure as described above provides a mechanism capable of further improving the quality of user experience.

### [BRIEF DESCRIPTION OF DRAWINGS]

[Figure 1] is a schematic diagram illustrating schematically a configuration example of an inhalation device.
[Figure 2] is an oblique view of an example of a heating system of an inhalation device according to an embodiment of the present disclosure.
[Figure 3] is an oblique view of the accommodating portion illustrated in Figure 2.
[Figure 4] is a cross-sectional view of the accommodating portion taken along the line 4-4 shown in Figure 3.
[Figure 5] is a cross-sectional view of the accommodating portion taken along the line 5-5 shown in Figure 4.
[Figure 6] is a longitudinal cross-sectional view of an accommodating portion including a non-pressing portion, in a state in which a stick-type substrate is held in a holding portion.
[Figure 7] is a longitudinal cross-sectional view of an accommodating portion including a pressing portion, in a state in which a stick-type substrate is held in a holding portion.
[Figure 8] is a cross-sectional view of the accommodating portion taken along the line 7-7 shown in Figure 7.
[Figure 9] is a diagram illustrating an example of the steps for manufacturing the heating system according to the same embodiment.
[Figure 10] is a diagram illustrating an example of the steps for manufacturing the heating system according to the same embodiment.
[Figure 11] is a diagram illustrating the configuration of an outer heat diffusion layer illustrated in Figure 10.
[Figure] 12 is a diagram illustrating the configuration of the heat insulating portion illustrated in Figure 10.
[Figure 13] illustrates an example of the steps for manufacturing the heating system according to a first modified example.
[Figure 14] illustrates an example of the steps for manufacturing the heating system according to a second modified example.
[Figure 15] illustrates an example of the steps for manufacturing the heating system according to a third modified example.
[Figure 16] illustrates an example of the steps for manufacturing the heating system according to a fourth modified example.

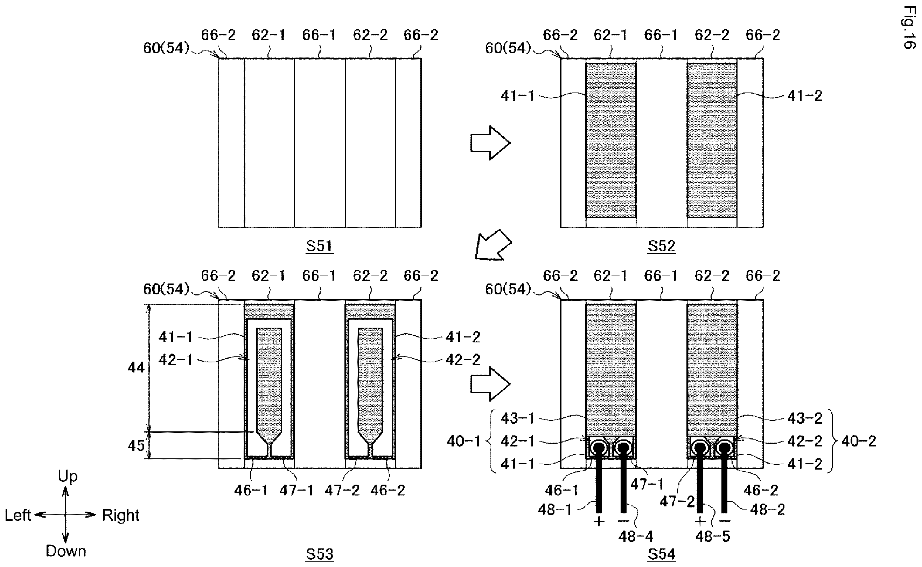

[Figure 16] illustrates an example of the steps for manufacturing the heating system according to a fifth modified example.

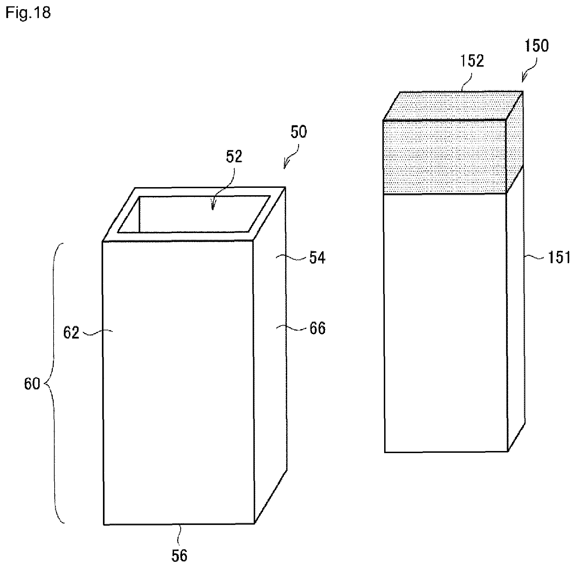

[Figure 18] is a diagram illustrating schematically an example of the configuration of an accommodating portion and a stick-type substrate according to a sixth modified example.

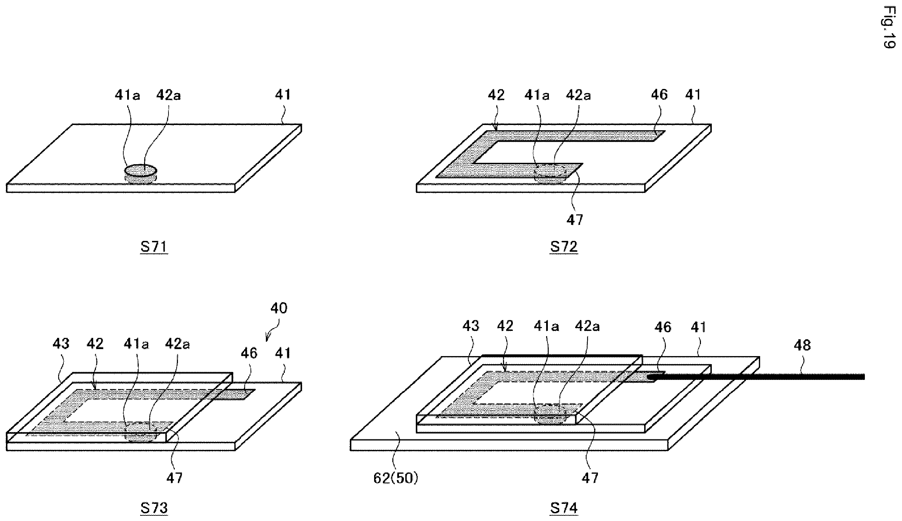

[Figure 19] illustrates an example of the steps for manufacturing the heating system according to a seventh modified example.

### [DESCRIPTION OF EMBODIMENTS]

Preferred embodiments of the present disclosure will now be described in detail with reference to the accompanying drawings. It should be noted that in the specification and the drawings, duplicate descriptions are omitted by using the same reference signs to denote constituent elements having substantially the same functional configuration.

In this specification and the drawings, elements having substantially identical functional configurations may also be distinguished by using the same code followed by an index comprising different alphabetic or numeric characters. For example, a plurality of elements having a substantially identical functional configuration are distinguished, as necessary, as devices 1-1, 1-2, and 1-3. However, if there is no need to specifically distinguish between each of the plurality of elements having a substantially identical functional configuration, only the same code is assigned. For example, devices 1-1, 1-2, and 1-3 are also simply referred to as device 1 when there is no need to distinguish between devices 1-1, 1-2, and 1-3.

### <1. Example configuration of inhalation device>

An inhalation device is a device for generating a substance to be inhaled by a user. Hereinafter, the substance generated by the inhalation device will be described as being an aerosol. Alternatively, the substance generated by the inhalation device may be a gas.

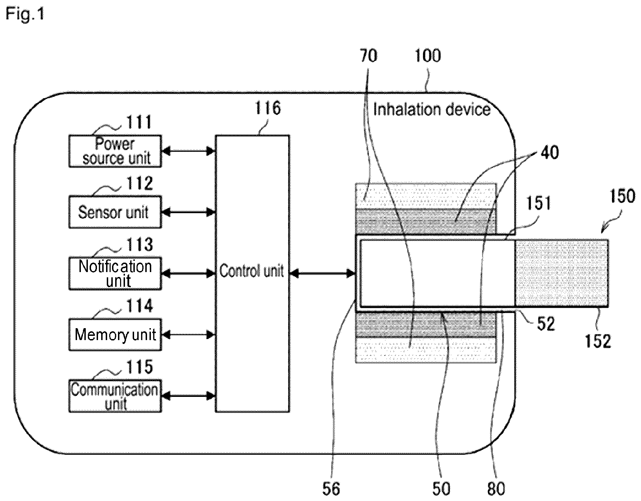

Figure 1 is a schematic diagram illustrating schematically a configuration example of an inhalation device. As illustrated in Figure 1, an inhalation device 100 according to the present configuration example comprises a power source unit 111, a sensor unit 112, a notification unit 113, a memory unit 114, a communication unit 115, a control unit 116, heating units 40, an accommodating unit 50, and a heat insulating portion 70.

The power source unit 111 stores electric power. The power source unit 111 then supplies the electric power to each component of the inhalation device 100 in accordance with control performed by the control unit 116. The power source unit 111 may be configured, for example, by a rechargeable battery such as a lithium ion secondary battery.

The sensor unit 112 acquires various types of information relating to the inhalation device 100. As an example, the sensor unit 112 is configured by a pressure sensor such as a condenser microphone, a flow rate sensor or a temperature sensor, etc., and acquires values associated with inhalation by a user. As another example, the sensor unit 112 is configured by an input device, such as a button or switch, for accepting input of information from the user.

The notification unit 113 notifies the user of the information. The notification unit 113 is configured by a light emitting device that emits light, a display device that displays images, a sound output device that outputs sound, or a vibrating device that vibrates, for example.

The memory unit 114 stores various types of information for the operation of the inhalation device 100. The memory unit 114 is configured by a non-volatile storage medium such as a flash memory, for example.

The communication unit 115 is a communication interface capable of performing communication conforming to any wired or wireless communication standard. Examples of communication standards that may be used include standards that employ Wi-Fi (registered trademark), Bluetooth (registered trademark), BLE (Bluetooth Low Energy) (registered trademark), NFC (Near-Field Communication), or LPWA (Low Power Wide Area), for example.

The control unit 116 functions as an arithmetic processing device and a control device, and controls overall operation within the inhalation device 100 in accordance with various programs. The control unit 116 is realized by a CPU (Central Processing Unit) or an electronic circuit such as a microprocessor, for example.

The accommodating portion 50 has an internal space 80, and holds a stick-type substrate 150 while accommodating a portion of the stick-type substrate 150 in the internal space 80. The accommodating portion 50 has an opening 52 allowing the internal space 80 to communicate with the outside, and accommodates the stick-type substrate 150 that has been inserted into the internal space 80 from the opening 52. For example, the accommodating portion 50 is a tubular body including the opening 52 and having a bottom wall 56 serving as a bottom surface, and defines the columnar internal space 80. An air flow path for supplying air to the internal space 80 may be connected to the accommodating portion 50. An air inflow hole, which is an inlet for air into the air flow path, is disposed in a side surface of the inhalation device 100, for example. An air outflow hole, which is an outlet for air from the air flow path to the internal space 80, is disposed in the bottom wall 56, for example.

The stick-type substrate 150 comprises a substrate portion 151 and a mouthpiece portion 152. The substrate portion 151 includes an aerosol source. The aerosol source includes a tobacco-derived or non-tobacco-derived flavor component. If the inhalation device 100 is a medical inhaler such as a nebulizer, the aerosol source may include a drug. The aerosol source may, for example, be a liquid such as water or a polyhydric alcohol, for example glycerol or propylene glycol, containing the tobacco-derived or non-tobacco-derived flavor component, or may be a solid including the tobacco-derived or non-tobacco-derived flavor component. In a state in which the stick-type substrate 150 is being held in the accommodating portion 50, at least a portion of the substrate portion 151 is accommodated in the internal space 80, and at least a portion of the mouthpiece portion 152 protrudes from the opening 52. Then, when the user holds the mouthpiece portion 152 protruding from the opening 52 in their mouth and inhales, air flows into the internal space 80 via the air flow path, which is not illustrated in the drawings, and reaches the inside of the user's mouth together with the aerosol generated from the substrate portion 151.

The heating units 40 heat the aerosol source to atomize the aerosol source, thereby generating the aerosol. In the example illustrated in Figure 1, the heating units 40 are configured in a film shape and are disposed so as to cover the outer periphery of the accommodating portion 50. Then, when the heating units 40 generate heat, the substrate portion 151 of the stick-type substrate 150 is heated from the outer periphery, generating the aerosol. The heating units 40 generate heat when supplied with electricity from the power source unit 111. By way of example, electricity may be supplied when the sensor unit 112 detects that the user has started inhaling and/or that predetermined information has been input. The supply of electricity may then be stopped when the sensor unit 112 detects that the user has finished inhaling and/or that predetermined information has been input.

The heat insulating portion 70 prevents heat transfer from the heating units 40 to other components. For example, the heat insulating portion 70 is configured from a vacuum heat insulating material or an aerogel heat insulating material, or the like.

A configuration example of the inhalation device 100 has been described above. The inhalation device 100 is, of course, not limited to the configuration described above, and may adopt various configurations, such as those illustrated below by way of example.

As an example, the accommodating portion 50 may include an opening and closing mechanism such as a hinge for opening and closing a portion of an outer shell that forms the internal space 80. Then, by opening and closing the outer shell, the accommodating portion 50 may clamp and accommodate the stick-type substrate 150 that has been inserted into the internal space 80. In this case, the heating units 40 may be provided on the clamping part of the accommodating portion 50, and may heat the stick-type substrate 150 while pressing the same.

Furthermore, the accommodating portion 50 may have a so-called counterflow air intake and exhaust configuration. In this case, air flows into the internal space 80 through the opening 52 as the user puffs. The air that has flowed in then passes through the interior of the stick-type substrate 150 from the tip of the stick-type substrate 150 and reaches the inside of the user's mouth together with the aerosol.

The stick-type substrate 150 is an example of an aerosol generating substrate that contains an aerosol source. The inhalation device 100 and the stick-type substrate 150 cooperate to generate an aerosol to be inhaled by the user. As such, the combination of the inhalation device 100 and the stick-type substrate 150 may be considered to be an aerosol generation system.

### <2. Technical features>

### <2. 1. Basic Configuration>

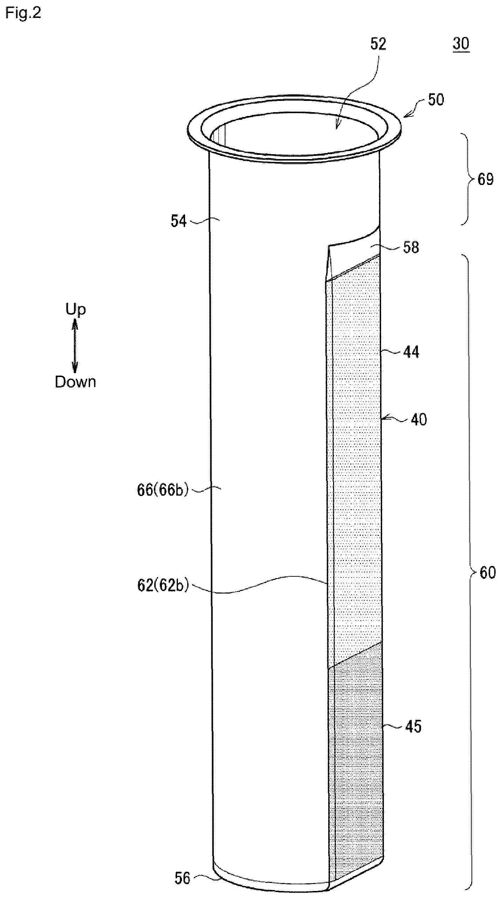

The basic configuration of the inhalation device 100 according to the present embodiment, as relates to the heating of the stick-type substrate 150, will now be described with reference to Figures 2 to 8.

Figure 2 is an oblique view of an example of a heating system 30 of the inhalation device 100 according to the present embodiment. The heating system 30 is a system of components involved in heating the stick-type substrate 150. The heating system 30 illustrated in Figure 2 comprises the heating units 40 and the accommodating portion 50. In addition to the heating units 40 and the accommodating portion 50 illustrated in Figure 2, the heating system 30 also includes an outer heat diffusion layer 90 and heat shrinkable tube 99, discussed hereinafter, and the heat insulating portion 70. As illustrated in Figure 2, the heating units 40 are disposed on the outer side of the accommodating portion 50. Therefore, when the heating units 40 generate heat, the accommodating portion 50 is heated from the outside and the stick-type substrate 150 is heated by heat transfer from the accommodating portion 50. This allows an aerosol to be generated from the stick-type substrate 150.

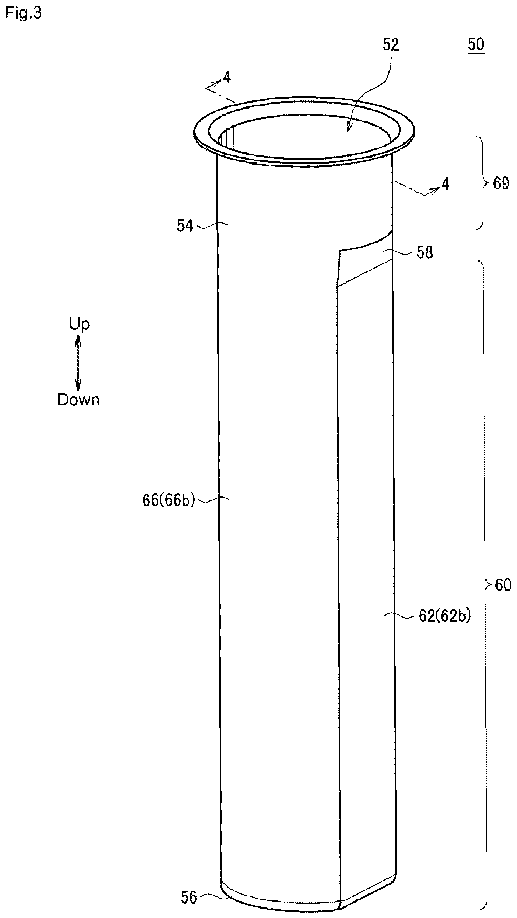

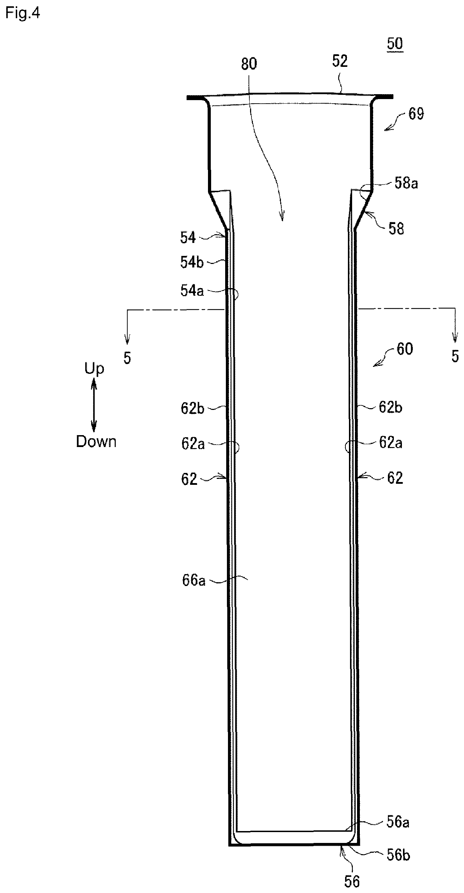

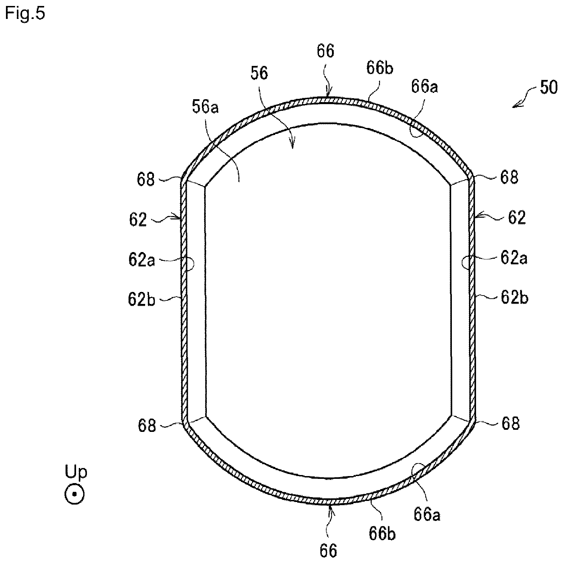

Figure 3 is an oblique view of the accommodating portion 50 illustrated in Figure 2. Figure 4 is a cross-sectional view of the accommodating portion 50 taken along the line 4-4 shown in Figure 3. Figure 5 is a cross-sectional view of the accommodating portion 50 taken along the line 5-5 shown in Figure 4. As illustrated in Figures 3 to 5, the accommodating portion 50 is a bottomed tubular body comprising the opening 52, a side wall 54 and the bottom wall 56, which blocks the end portion on the opposite side to the opening 52. The side wall 54 has an inner surface 54a and an outer surface 54b. The bottom wall 56 has an inner surface 56a and an outer surface 56b. The stick-type substrate 150 is inserted into the accommodating portion 50 through the opening 52 and is accommodated in the internal space 80 surrounded by the side wall 54 and the bottom wall 56. The accommodating portion 50 is preferably made of a metal having a high thermal conductivity and may, for example, be made of SUS (steel use stainless) or the like. This allows efficient heating of the stick-type substrate 150.

The stick-type substrate 150 is inserted and removed along the axial direction of the accommodating portion 50, which is a tubular body. Among the axial directions, the direction in which the stick-type substrate 150 is inserted is also referred to as "down", and the direction in which the stick-type substrate 150 is withdrawn is also referred to as "up". The axial direction is also referred to as the up-down direction. The up-down direction may be the longitudinal direction of the accommodating portion 50. Among the directions perpendicular to the up-down direction, the direction toward the central axis of the accommodating portion 50 is also referred to as inward and the direction moving away from the central axis is also referred to as outward.

As illustrated in Figures 3 to 5, the accommodating portion 50 has a holding portion 60 that holds the stick-type substrate 150. The holding portion 60 includes pressing portions 62 that press a portion of the stick-type substrate 150, and non-pressing portions 66. The pressing portions 62 have an inner surface 62a and an outer surface 62b. The non-pressing portions 66 have an inner surface 66a and an outer surface 66b. The pressing portions 62 and the non-pressing portions 66 are portions of the side wall 54 of the accommodating portion 50. The pressing portions 62 are an example of first side walls. The non-pressing portions 66 are an example of second side walls that are different from the first side walls.

The opening 52 of the accommodating portion 50 can preferably accept the stick-type substrate 150 without applying pressure thereto. In other words, the opening 52 of the accommodating portion 50 is preferably configured to be larger than the stick-type substrate 150 in a plane perpendicular to the up-down direction. The shape of the opening 52 of the accommodating portion 50 in a plane perpendicular to the up-down direction may be polygonal or oval, but is preferably circular.

As illustrated in Figure 2, the heating units 40 are disposed on the outer surfaces 62b of the pressing portions 62. The heating units 40 are preferably disposed on the outer surface 62b of the pressing portions 62 without a gap. Furthermore, the heating units 40 are preferably disposed over the entire outer surface 62b of the pressing portions 62. However, the heating units 40 are preferably disposed so as not to protrude beyond the outer surface 62b of the pressing portions 62. Of course, the heating units 40 may be disposed so as to protrude from the outer surface 62b of the pressing portions 62 onto the outer surface 66b of the non-pressing portions 66.

As illustrated in Figure 2, the heating units 40 each have a heat generating region 44 and a non-heat generating region 45. The heat generating regions 44 are regions that generate heat when an electric current is applied to the heating units 40. The non-heat generating regions 45 are regions that do not generate heat or generate very little heat even when an electric current is applied to the heating units 40. The heat generating regions 44 are disposed on the outer surface 62b of the pressing portions 62. With this configuration, it is possible to heat the stick-type substrate 150 efficiently while pressing the stick-type substrate 150 with the pressing portions 62.

As illustrated in Figures 3 to 5, in the present embodiment, the accommodating portion 50 has two pressing portions 62 and two non-pressing portions 66. Furthermore, the pressing portions 62 and the non-pressing portions 66 are arranged alternately along the circumferential direction of the accommodating portion 50. In particular, the two pressing portions 62 of the holding portion 60 oppose one another. The distance between the inner surfaces 62a of the two pressing portions 62 is, at least partially, less than the width of the part of the stick-type substrate 150 that is disposed between the pressing portions 62 when inserted into the accommodating portion 50. With this configuration, the stick-type substrate 150 can be pressed by the two opposing pressing portions 62.

As illustrated in Figures 3 to 5, the inner surfaces 66a of the non-pressing portions 66 of the holding portion 60 are curved in a plane perpendicular to the longitudinal direction of the accommodating portion 50. Preferably, the shape of the inner surfaces 66a of the non-pressing portions 66 in a plane perpendicular to the longitudinal direction of the accommodating portion 50 is identical to the shape of the opening 52 in the plane perpendicular to the longitudinal direction of the accommodating portion 50 at any position in the longitudinal direction of the accommodating portion 50. In other words, the inner surfaces 66a of the non-pressing portions 66 are preferably formed by extending the inner surface of the accommodating portion 50 that forms the opening 52 in the longitudinal direction. The outer surfaces 66b of the non-pressing portions 66 of the holding portion 60 are curved parallel to the inner surfaces 66a.

As illustrated in Figure 5, the inner surfaces 62a of the pressing portions 62 comprise a pair of opposing planar pressing surfaces having a planar shape. Meanwhile, the inner surfaces 66a of the non-pressing portions 66 connect both ends of the pair of planar pressing surfaces and comprise a pair of opposing curved non-pressing surfaces having a curved surface shape. As illustrated in the drawings, the curved non-pressing surfaces may have an overall arc-shaped cross-section in a plane perpendicular to the longitudinal direction of the accommodating portion 50. The outer surfaces 62b of the pressing portions 62 and the outer surfaces 66b of the non-pressing portions 66 may be connected to one another at an angle, and boundaries 68 may be formed between the outer surfaces 62b of the pressing portions 62 and the outer surfaces 66b of the non-pressing portions 66. As illustrated in Figure 5, the pressing portions 62 and the non-pressing portions 66 (i.e. the side wall 54 of the accommodating portion 50) may have a uniform thickness. For example, the pressing portions 62 may comprise a flat plate. In addition, the non-pressing portions 66 may comprise a curved plate that curves to the outside of the accommodating portion 50 along the circumferential direction of the accommodating portion 50.

As illustrated in Figures 3 and 4, the accommodating portion 50 preferably has first guide portions 58 having a tapered surface 58a that connects the inner surface of the accommodating portion 50 (i.e. a non-holding portion 69) forming the opening 52 and the inner surfaces 62a of the pressing portions 62. The first guide portions 58 provide a smooth connection between the pressing portions 62 and the non-holding portion 69, thereby allowing the stick-type substrate 150 to be suitably guided into the holding portion 60 in the process of the stick-type substrate 150 being inserted into the accommodating portion 50.

As illustrated in Figure 4, the accommodating portion 50 preferably has a tubular non-holding portion 69 between the opening 52 and the holding portion 60. The non-holding portion 69 is a part of the accommodating portion 50 that does not contribute to holding the stick-type substrate 150. For example, in a plane perpendicular to the longitudinal direction of the accommodating portion 50, the non-holding portion 69 may be formed to be larger than the stick-type substrate 150. This allows for easy insertion of the stick-type substrate 150 into the accommodating portion 50.

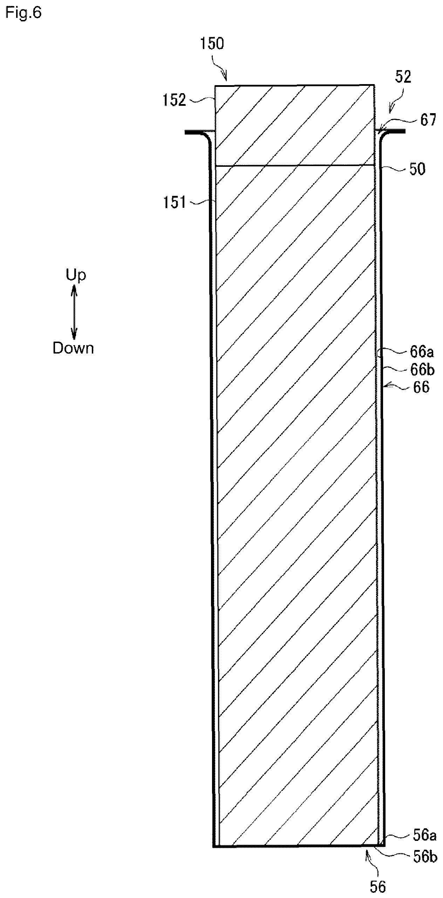

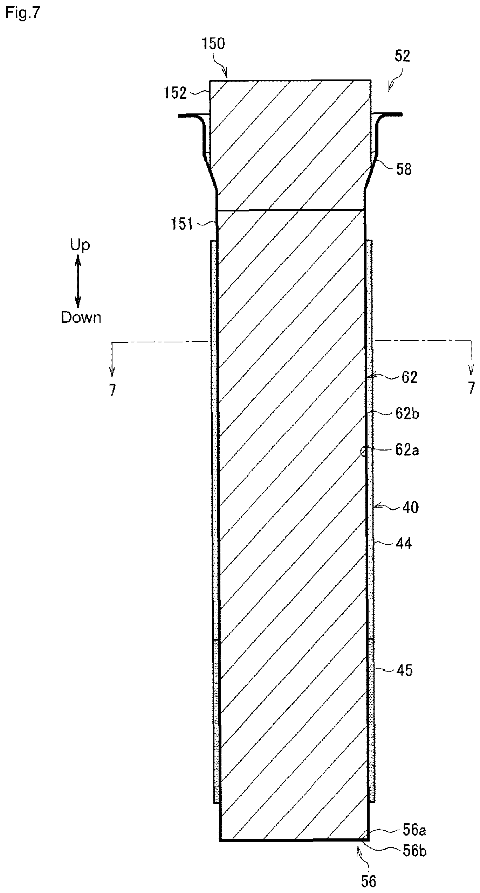

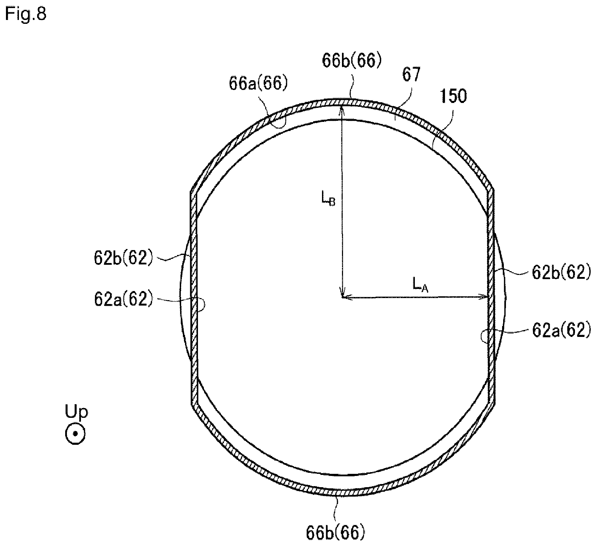

Figure 6 is a longitudinal cross-sectional view of the accommodating portion 50 including the non-pressing portions 66, in a state in which the stick-type substrate 150 is being held by the holding portion 60. Figure 7 is a longitudinal cross-sectional view of the accommodating portion 50 including the pressing portions 62, in a state in which the stick-type substrate 150 is being held by the holding portion 60. Figure 8 is a cross-sectional view of the accommodating portion 50 taken along the line 7-7 shown in Figure 7. It should be noted that, in Figure 8, a cross-section through the stick-type substrate 150 in the state before being pressed is shown in order to make it easy to recognize that the stick-type substrate 150 is pressed by the pressing portions 62.

As illustrated in Figure 6, the stick-type substrate 150 is pressed by the pressing portions 66, and the inner surfaces 66a of the pressing portions 66 and the stick-type substrate 150 are in close contact with one another. Meanwhile, as illustrated in Figure 7, a gap 67 is formed between the inner surfaces 66a of the non-pressing portions 66 and the stick-type substrate 150.

As illustrated in Figure 8, the gap 67 between the inner surfaces 66a of the non-pressing portions 66 and the stick-type substrate 150 is substantially maintained even when the stick-type substrate 150 is held by the holding portion 60 and the stick-type substrate 150 is pressed and deformed by the pressing portions 62. If the accommodating portion 50 has a counterflow air intake and exhaust configuration, the gap 67 can form an air flow path that provides communication between the opening 52 and the tip of the stick-type substrate 150.

As illustrated in Figure 8, in a state in which the stick-type substrate 150 is being held by the holding portion 60, a distance L_{A} between the inner surface 62a of the pressing portions 62 and the center of the stick-type substrate 150 is less than a distance L_{B} between the inner surface 66a of the non-pressing portions 66 and the center of the stick-type substrate 150. With this configuration, the distance between the heating units 40 disposed on the outer surface 62b of the pressing portions 62 and the center of the stick-type substrate 150 can be reduced compared to a case in which the pressing portions 62 are not provided. The stick-type substrate 150 heating efficiency can thus be improved.

As illustrated in Figures 3 to 8, the outer peripheral surface of the holding portion 60 preferably has the same shape and size (the outer peripheral length of the holding portion 60 in a plane perpendicular to the longitudinal direction of the holding portion 60) along the entire longitudinal length of the holding portion 60. This makes it possible to ensure the gap 67 while uniformly pressing the stick-type substrate 150, over the entire holding portion 60 in the up-down direction.

As described hereinabove, the inhalation device 100 according to the present embodiment holds and heats the stick-type substrate 150 while pressing the same by means of the pressing portions 62. This configuration makes it possible to improve the stick-type substrate 150 heating efficiency compared to a case in which the stick-type substrate 150 is heated without being pressed.

### <2. 2. Configuration of heating system 30>

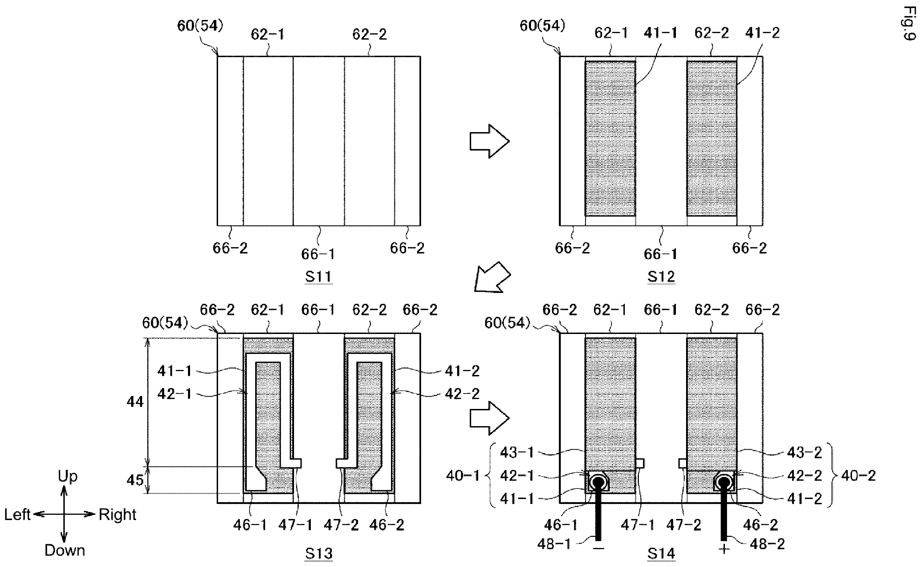

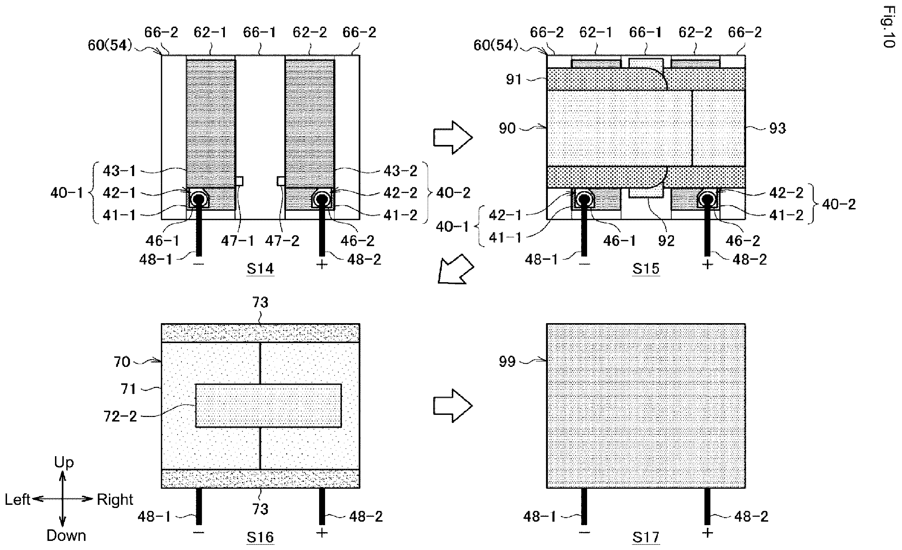

The heating system 30 according to the present embodiment is manufactured by laminating the components constituting the heating system 30 sequentially onto the outer side of the side wall 54 of the accommodating portion 50. The configuration of the heating system 30 will now be described while describing the manufacturing process of the heating system 30 with reference to Figures 9 and 10.

Figures 9 and 10 are drawings illustrating an example of a process for manufacturing the heating system 30 according to the present embodiment. The process for manufacturing the heating system 30 according to the present embodiment proceeds sequentially through manufacturing steps S11 to S17 illustrated in Figures 9 and 10. In the following, the two pressing portions 62 of the holding portion 60 are in some cases distinguished as the pressing portion 62-1 and the pressing portion 62-2. Similarly, the two non-pressing portions 66 of the holding portion 60 are in some cases distinguished as the non-pressing portion 66-1 and the non-pressing portion 66-2. In Figures 9 and 10, each manufacturing step is illustrated on an unfolded view in which the side wall 54 of the accommodating portion 50 (in particular, the part corresponding to the holding portion 60) is divided at the center of the non-pressing portion 66-2 and is unfolded. The left-right direction in the unfolded views corresponds to the circumferential direction of the accommodating portion 50.

In manufacturing step S11 of Figure 9, the accommodating portion 50 is illustrated in a state before the other components have been laminated onto the holding portion 60.

In manufacturing step S12 of Figure 9, first electrically insulating layers 41 (41-1 and 41-2) are first laminated onto the pressing portion 62. Specifically, the first electrically insulating layer 41-1 is laminated onto the outer side of the pressing portion 62-1, and the first electrically insulating layer 41-2 is laminated onto the outer side of the pressing portion 62-2. The first electrically insulating layer 41 is made of an electrically insulating material. Examples of materials that can be used to form the first electrically insulating layer 41 include glass and ceramic, for example. The first electrically insulating layers 41 are laminated using a vapor deposition process or a printing process. A vapor deposition process is a process in which a substance is vaporized toward the surface of a target object to form a thin film coating. A printing process is a process in which a liquid is ejected toward the surface of the target object to form a thin film coating.

In manufacturing step S13 of Figure 9, resistive heating layers 42 (42-1 and 42-2) are laminated onto the outer sides of the pressing portions 62 of the partially manufactured heating system 30 that has passed through manufacturing step S12. Specifically, the resistive heating layer 42-1 is laminated onto the outer side of the first electrically insulating layer 41-1 laminated onto the pressing portion 62-1, and the resistive heating layer 42-2 is laminated onto the outer side of the first electrically insulating layer 41-2 laminated onto the pressing portion 62-2. In particular, the resistive heating layers 42 are laminated onto the first electrically insulating layers 41 in the shape of a single line that moves back and forth in the up-down direction while leaving a gap in the left-right direction. The resistive heating layers 42 are made of an electrically conductive material. Examples of materials that can be used to form the resistive heating layers 42 include metallic materials such as SUS and non-metallic materials such as silicon carbide. The resistive heating layers 42 may also be made of an electrically conductive paste-like material. An example of such a material is a material in which a main constituent comprising silver is mixed with a resistance adjusting agent. When an electric current is applied to the resistive heating layers 42, Joule heat corresponding to the electrical resistance is emitted. The resistive heating layers 42 are laminated using a vapor deposition process or a printing process.

Here, as illustrated in Figure 9, the resistive heating layer 42-1 forms an open circuit having a first end portion 46-1 and a second end portion 47-1 as the two ends thereof. The resistive heating layer 42-2 also forms an open circuit having a first end portion 46-2 and a second end portion 47-2 as the two ends thereof. The first end portions 46 (46-1 and 46-2) are disposed within the first electrically insulating layers 41. In particular, the first end portions 46 are disposed in lower end portions of the first electrically insulating layers 41. Meanwhile, the second end portions 47 (47-1 and 47-2) are disposed protruding from the first electrically insulating layers 41. In particular, the second end portions 47 protrude from the first electrically insulating layers 41, further protrude from the pressing portions 62, and are disposed in the non-pressing portion 66.

In manufacturing step S14 of Figure 9, second electrically insulating layers 43 (43-1 and 43-2) are laminated onto the outer sides of the pressing portions 62 of the partially manufactured heating system 30 that has passed through manufacturing step S13. Specifically, a second electrically insulating layer 43-1 is laminated onto the outside of the first electrically insulating layer 41-1 and the resistive heating layer 42-2 that are laminated onto the pressing portion 62-1, and a second electrically insulating layer 43-2 is laminated onto the outside the first electrically insulating layer 41-2 and the resistive heating layer 42-2 that are laminated onto the pressing portion 62-2. Similarly to the first electrically insulating layers 41, the second electrically insulating layers 43 are made of an electrically insulating material. The second electrically insulating layers 43 are laminated using a vapor deposition process or a printing process.

Further, in manufacturing step S14, a conducting wire 48-1 is connected to the resistive heating layer 42-1 and a conducting wire 48-2 is connected to the resistive heating layer 42-2. Specifically, the conducting wire 48-1 is connected to the first end portion 46-1 of the resistive heating layer 42-1 and the conducting wire 48-2 is connected to the first end portion 46-2 of the resistive heating layer 42-2. The conducting wires 48 (48-1 and 48-2) are connected to the power source unit 111. As an example, the first end portion 46-1 of the resistive heating layer 42-1 is connected to the negative electrode of the power source unit portion 111 via the conducting wire 48-1. Meanwhile, the first end portion 46-2 of the resistive heating layer 42-2 is connected to the positive electrode of the power source unit 111 via the conducting wire 48-2. The power source unit 111 then supplies electric power to the resistive heating layers 42 on the basis of control by the control unit 116, causing the resistive heating layers 42 to generate heat.

Here, the accommodating portion 50 is made of an electrically conductive material. An example of a material that can be used to form the accommodating portion 50 is SUS.

The second end portion 47-1 of the resistive heating layer 42-1 protrudes from the first electrically insulating layer 41-1 and is connected to the accommodating portion 50, and is electrically connected to the power source unit 111 via the accommodating portion 50. Similarly, the second end portion 47-2 of the resistive heating layer 42-2 protrudes from the first electrically insulating layer 41-2 and is connected to the accommodating portion 50, and is electrically connected to the power source unit 111 via the accommodating portion 50. More specifically, the second end portion 47-1 of the resistive heating layer 42-1 and the second end portion 47-2 of the resistive heating layer 42-2 adjacent to the resistive heating layer 42-1 are electrically connected via the accommodating portion 50. Then, the first end portion 46-1 of the resistive heating layer 42-1 is electrically connected to the power source unit 111 via the conducting wire 48-1, and the first end portion 46-2 of the resistive heating layer 42-2 is electrically connected to the power source unit 111 via the conducting wire 48-2. With the configuration described hereinabove, the conducting wire 48-1, the resistive heating layer 42-1, the accommodating portion 50, the resistive heating layer 42-2, and the conducting wire 48-2 form one series circuit that is connected to the power source unit 111. When the power source unit 111 supplies electric power to this series circuit, heat can be generated in the resistive heating layer 42-1 and the resistive heating layer 42-2.

The first electrically insulating layer 41-1, the resistive heating layer 42-1 and the second electrically insulating layer 43-1 described hereinabove constitute a heating unit 40-1. Further, the first electrically insulating layer 41-2, the resistive heating layer 42-2 and the second electrically insulating layer 43-2 constitute a heating unit 40-2. Here, each component constituting the heating units 40 (40-1 and 40-2) is laminated using a printing process or a vapor deposition process. The occurrence of defects such as misalignment and peeling of the heating units 40 can thus be prevented, and consequently the manufacturing accuracy of the heating system 30 can be improved in comparison with other manufacturing methods such as a method in which the heating units 40 are manufactured separately and are bonded to the accommodating portion 50. As a result, it is possible to improve the stick-type substrate 150 heating efficiency, thereby improving the quality of the user experience.

Supplementary information regarding the features of the heating units 40 will now be provided.

Referring again to manufacturing steps S12 to S14, the first electrically insulating layer 41-1 is laminated inward of the resistive heating layer 42-1, and the second electrically insulating layer 43-1 is laminated outward of the resistive heating layer 42-1. Furthermore, at least a portion of the resistive heating layer 42-1 is sandwiched between the first electrically insulating layer 41-1 and the resistive heating layer 42-2. With this configuration, it is possible to prevent a short circuit within the resistive heating layer 42-1 via a component on the inner side of the heating units 40 (for example, the accommodating portion 50) or a component on the outer side of the heating units 40 (for example, an outer heat diffusion layer g1, discussed hereinafter). The same applies to the first electrically insulating layer 41-2, the resistive heating layer 42-2 and the second electrically insulating layer 43-2.

Referring again to manufacturing step S13, the resistive heating layer 42-1 and the resistive heating layer 42-2 are laminated onto the outer sides of the pressing portion 62-1 and the pressing portion 62-2 adjacent to and on both sides of the non-pressing portion 66-1, in a state separated from one another at the non-pressing portions 66-1. With this configuration, the resistive heating layers 42 can be disposed on the flat surfaces on the pressing portions 62. The occurrence of defects such as misalignment and peeling can thus be prevented, and consequently the manufacturing accuracy of the heating system 30 can be improved in comparison with a case in which the resistive heating layers 42 are disposed on the curved surfaces on the non-pressing portions 66. As a result, it is possible to improve the stick-type substrate 150 heating efficiency, thereby improving the quality of the user experience.

Referring again to manufacturing step S13, the second end portion 47-1 of the resistive heating layer 42-1 that protrudes from the first electrically insulating layer 41-1 protrudes from the pressing portion 62-1 and is connected to the non-pressing portion 66-1. Meanwhile, the second end portion 47-2 of the resistive heating layer 42-2 that protrudes from the first electrically insulating layer 41-2 protrudes from the pressing portion 62-2 and is connected to the non-pressing portion 66-1. That is, the second end portion 47-1 of the resistive heating layer 42-1 and the second end portion 47-2 of the resistive heating layer 42-2 are disposed protruding in directions that approach one another from the left and right ends of the non-pressing portion 66-1. With this configuration, the distance between the second end portion 47-1 of the resistive heating layer 42-1 and the second end portion 47-2 of the resistive heating layer 42-2 can be minimized. As a result, it is possible to facilitate the conduction of electricity between the resistive heating layer 42-1 and the resistive heating layer 42-2.

Referring again to manufacturing step S13, the resistive heating layers 42 laminated in the heat generating region 44 are configured to be thin. This allows the electrical resistance of the resistive heating layers 42 laminated in the heat generating region 44 to be increased to generate a high Joule heat when electric power is applied. Meanwhile, the resistive heating layers 42 laminated in the non-heat generating regions 45 of the heating units 40 are configured to be wider than the resistive heating layers 42 laminated in the heat generating region 44. This allows the electrical resistance of the resistive heating layers 42 laminated in the non-heat generating regions 45 to be reduced so that no Joule heat or only a very small amount of Joule heat is generated when electric power is applied.

Referring again to manufacturing step S14, the first end portions 46 to which the conducting wires 48 are connected are configured in the resistive heating layers 42 in the non-heat generating regions 45, which are configured to be wider than the resistive heating layers 42 in the heat generating regions 44. This makes it possible to prevent heat transfer to the conducting wires 48 and to prevent the connecting parts between the conducting wires 48 and the resistive heating layers 42 from being damaged by heat.

Referring again to manufacturing step S14, the conducting wires 48 are only connected at one of the two ends of each resistive heating layer 42. With this configuration, the number of conducting wires 48 can be reduced in comparison with a case in which conducting wires 48 are connected to both ends of the resistive heating layers 42. This makes it possible to inhibit the occurrence of poor connections between the conducting wires 48 and the resistive heating layers 42, thereby improving the quality of the user experience.

The resistive heating layers 42 are disposed in positions corresponding to the substrate portion 151, in which the aerosol source is distributed, of the stick-type substrate 150 accommodated in the accommodating portion 50. Specifically, in a state in which the stick-type substrate 150 is accommodated in the accommodating portion 50, as illustrated in Figure 7, the heat generating region 44 in which the resistive heating layers 42 are laminated is disposed in a position within the pressing portion 62 corresponding to the substrate portion 151. With this configuration, it is possible to improve the stick-type substrate 150 heating efficiency.

It is desirable that the part of the outer periphery of the accommodating portion 50 on which the first electrically insulating layers 41 are laminated occupies less than 50% of the outer periphery of the accommodating portion 50. More simply, it is desirable that the pressing portions 62 occupy less than 50% of the outer periphery of the accommodating portion 50. With this configuration, the area of the heat generating region 44 can be reduced to increase the watt density. As a result, it is possible to improve the stick-type substrate 150 heating efficiency.

The control unit 116 may control the temperature to which the stick-type substrate 150 is heated by estimating and controlling the temperature of the resistive heating layers 42 on the basis of the electrical resistance value of the resistive heating layers 42. The electrical resistance value of the resistive heating layers 42 is measured on the basis of the amount of voltage drop between the conducting wire 48-1 and the conducting wire 48-2. In the present embodiment, it is considered that the temperature of the resistive heating layers 42 can be estimated to be a temperature close to the temperature of the accommodating portion 50, to the extent that the resistive heating layer 42-1 and the resistive heating layer 42-2 are electrically connected via the accommodating portion 50. In consideration of the fact that the stick-type substrate 150 is heated directly by the accommodating portion 50, this configuration makes it possible for temperature control of the stick-type substrate 150 to be implemented more suitably, thereby improving the quality of the user experience.

Supplementary information regarding the features of the heating units 40 has been provided above. The subsequent manufacturing steps will next be described with reference to Figure 10.

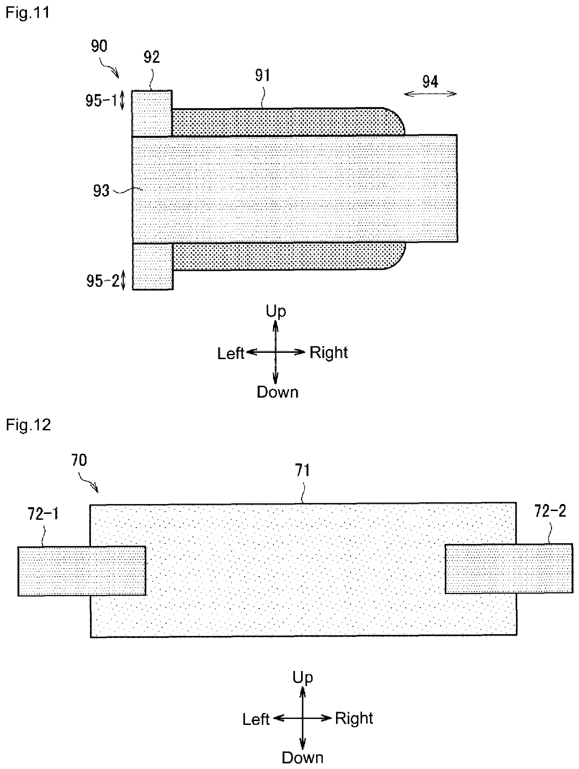

In manufacturing step S15 of Figure 10, the outer heat diffusion layer 90 is laminated onto the outer side of the partially manufactured heating system 30 that has passed through manufacturing step S14. Specifically, the outer heat diffusion layer 90 is wrapped around and laminated onto the outer side of the side wall 54 of the accommodating portion 50, outward of the heating unit 40. The outer heat diffusion layer 90 is an example of a second heat diffusion layer that diffuses the heat of the heating units 40 on the outer side of the heating units 40. With this configuration, the heat of the heating units 40 laminated onto the pressing portions 62 can be diffused throughout the entire accommodating portion 50 including the non-pressing portions 66. As a result, the stick-type substrate 150 accommodated in the accommodating portion 50 can be heated efficiently. The configuration of the outer heat diffusion layer 90 will be described with reference to Figure 11.

Figure 11 is a diagram illustrating the configuration of the outer heat diffusion layer 90 illustrated in Figure 10. As illustrated in Figure 11, the outer heat diffusion layer 90 comprises a graphite sheet 91, a vertically long PI tape 92 and a horizontally long PI tape 93.

The graphite sheet 91 is a sheet-like member made of graphite. The thermal conductivity of the graphite sheet 91 is at least higher than the thermal conductivity of the accommodating portion 50. With this configuration, the graphite sheet 91 is capable of efficiently diffusing the heat of the heating unit 40. It should be noted that a sheet-like member made of silicon or acrylic, for example, may be used instead of the graphite sheet 91.

The vertically long PI tape 92 and the horizontally long PI tape 93 are formed by applying an adhesive to one surface of a film-like member made of PI (Polyimide). The tensile strength of the vertically long PI tape 92 and the horizontally long PI tape 93 is higher than the tensile strength of the graphite sheet 91. Consequently, the vertically long PI tape 92 and the horizontally long PI tape 93 can prevent tearing of the graphite sheet 91 while securing the graphite sheet 91 around the periphery of the accommodating portion 50.

The outer heat diffusion layer 90 is formed by bonding the graphite sheet 91 as a lowermost layer, the vertically long PI tape 92 as a middle layer, and the horizontally long PI tape 93 as an uppermost layer, in an overlapping state. The vertically long PI tape 92 and the horizontally long PI tape 93 are overlapped in a state in which the adhesive surfaces thereof face the lowermost layer. Here, it should be noted that the layer that will be on the inner side when the outer heat diffusion layer 90 is wrapped around the accommodating portion 50 is defined as the lowermost layer, and the layer that will be on the outer side is defined as the uppermost layer. Then, in manufacturing step S15 illustrated in Figure 10, the outer heat diffusion layer 90 is wrapped and disposed so as to cover the outer side of the heating units 40 disposed on the outer side of the accommodating portion 50, with the graphite sheet 91 on the inner side and the horizontally long PI tape 93 on the outer side. With this configuration, it is possible for the graphite sheet 91 to be brought into close contact with the heating units 40 or the accommodating portion 50. As a result, it is possible to improve the effect of thermal diffusion from the heating units 40 to the accommodating portion 50 via the graphite sheet 91. In addition, with this configuration, the graphite sheet 91, which is in close contact with the heating units 40 or the accommodating portion 50, can be protected from the outside by the horizontally long PI tape 93. As a result, it is possible to improve the effect of the horizontally long PI tape 93 preventing the graphite sheet 91 from being torn.

Here, it is desirable that the graphite sheet 91 is laminated so as to overlap the heat generating region 44 of the heating units 40. With this configuration, the heat from the heating units 40 can be efficiently diffused. Meanwhile, it is desirable that the graphite sheet 91 is laminated so as to avoid the non-heat generating regions 45 of the heating units 40. With this configuration, it is possible to prevent heat transfer to the conducting wires 48 and to prevent the connecting parts between the conducting wires 48 and the resistive heating layers 42 from being damaged by heat.

The graphite sheet 91 is formed to be longer than the outer periphery of the accommodating portion 50 (in particular the holding portion 60) in the left-right direction. As a result, the graphite sheet 91 is wrapped one or more times around the outer surface of the accommodating portion 50. With this configuration, the graphite sheet 91 completely covers the outer periphery of the accommodating portion 50, allowing the heat of the heating units 40 to be diffused throughout the entire accommodating portion 50.

As illustrated in Figure 11, the vertically long PI tape 92 is formed to be longer than the graphite sheet 91 in the up-down direction and is positioned such that both ends in the up-down direction protrude from the graphite sheet 91. Then, referring again to manufacturing step S15 of Figure 10, these protruding parts 95-1 and 95-2 are directly bonded to the non-pressing portions 66 in which the heating units 40 are not disposed. With this configuration, it is possible to secure the outer heat diffusion layer 90 firmly to the accommodating portion 50 to prevent misalignment of the outer heat diffusion layer 90. It is also possible to reduce the load on the heating units 40 when wrapping the outer heat diffusion layer 90 and prevent damage to the heating units 40 in comparison with a case in which the vertically long PI tape 92 is bonded to the heating units 40 on the pressing portions 62.

As illustrated in Figure 11, the horizontally long PI tape 93 is formed to be longer than the graphite sheet 91 in the left-right direction and is positioned such that the right end portion thereof protrudes from the graphite sheet 91. Then, referring again to manufacturing step S15 of Figure 10, this protruding part 94 is bonded to the horizontally long PI tape 93 wrapped one turn inward of the protruding part 94. With this configuration, the position of the graphite sheet 91 can be firmly secured by means of the horizontally long PI tape 93. As a result, it is possible to prevent a situation in which an unnecessary force is applied to the graphite sheet, causing the graphite sheet 91 to break.

Next, in manufacturing step S16 of Figure 10, the heat insulating portion 70 is laminated onto the outer side of the partially manufactured heating system 30 that has passed through manufacturing step S15. Specifically, the heat insulating portion 70 is wrapped around and laminated onto the outer side of the side wall 54 of the accommodating portion 50, outward of the heating units 40 and the outer heat diffusion layer 90. The heat insulating portion 70 is an example of a heat insulating layer that blocks the heat of the heating units 40. With such a configuration, the heat of the heating units 40 can be prevented from diffusing to the outside. As a result, it is possible to prevent the occurrence of defects such as malfunctions of electronic circuits caused by high temperatures. Here, the heat insulating portion 70 is laminated so as to cover a portion, in the up-down direction, of the side wall 54 of the accommodating portion 50 in the up-down direction. It is desirable that the heat insulating portion 70 completely covers the heat generating region 44 of the heating units 40 and the outer heat diffusion layer 90. Meanwhile, end portions of the heat insulating portion 70 in the up-down direction and parts of the side wall 54 of the accommodating portion 50 that are exposed from the heat insulating portion 70 are sealed by means of sealing members 73. The sealing members 73 are made of a material having a prescribed heat resistance, such as silicon. With this configuration, the heat insulating effect of the heat insulating portion 70 can be improved. The configuration of the heat insulating portion 70 will be described with reference to Figure 12.

Figure 12 is a drawing illustrating the configuration of the heat insulating portion 70 illustrated in Figure 10. As illustrated in Figure 12, the heat insulating portion 70 is configured by laminating a heat insulating sheet 71 and PI tapes 72 (72-1 and 72-2). The heat insulating sheet 71 is a member that blocks heat. For example, the heat insulating sheet 71 is made of a glass material, a vacuum heat insulating material, an aerogel heat insulating material, or the like. The PI tapes 72 are tapes made of PI. The PI tapes 72 are formed by applying an adhesive to one surface of a film-like member made of PI. Then, in manufacturing step S16 illustrated in Figure 10, the heat insulating portion 70 is wrapped and disposed so as to cover the outer side of the outer heat diffusion layer 90 disposed on the outer side of the accommodating portion 50, with the heat insulating sheet 71 on the inner side and the PI tapes 72 on the outer side, and with the adhesive surface of the PI tapes 72 facing inward. With this configuration, it is possible for the heat insulating sheet 71 to be brought into close contact with the outer heat diffusion layer 90. As a result, the heat insulating effect of the heat insulating sheet 71 can be improved.

The heat insulating sheet 71 is formed to be longer than the graphite sheet 91 in the up-down direction, and is positioned such that end portions of the heat insulating sheet 71 in the up-down direction protrude beyond the graphite sheet 91. With this configuration, the heat insulating sheet 71 can completely cover the graphite sheet 91 in the up-down direction. Furthermore, the heat insulating sheet 71 is formed to be longer than the outer periphery of the accommodating portion 50 (in particular the holding portion 60) in the left-right direction. As a result, the heat insulating sheet 71 is wrapped one or more times around the outer surface of the accommodating portion 50. With this configuration, the outer periphery of the accommodating portion 50 can be completely covered by the heat insulating sheet 71. This makes it possible to prevent the heat from the heating units 40 that is diffused by the outer heat diffusion layer 90 from diffusing further outward than the heat insulating portion 70.

The PI tape 72-1 is positioned on the left end portion of the heat insulating sheet 71 so that approximately half of the PI tape 72-1 protrudes toward the left from the heat insulating sheet 71. Then, the PI tape 72-1 is bonded to the outer heat diffusion layer 90 (for example, the horizontally long PI tape 93) wrapped around the holding portion 60. With this configuration, it is possible to fix the position of the heat insulating portion 70 to prevent misalignment of the heat insulating portion 70.

The PI tape 72-2 is positioned on the right end portion of the heat insulating sheet 71 so that approximately half of the PI tape 72-2 protrudes toward the right from the heat insulating sheet 71. Then, the protruding part of the PI tape 72-2 is bonded to the heat insulating portion 70 (for example, the heat insulating sheet 71) wrapped one turn inward of the protruding part. With this configuration, it is possible to fix the position of the heat insulating portion 70 to prevent misalignment of the heat insulating portion 70.

In manufacturing step S17 of Figure 10, a heat shrinkable tube 99 is laminated onto the outer side of the partially manufactured heating system 30 that has passed through manufacturing step S16. The heat shrinkable tube 99 is a tubular member that shrinks upon application of heat. For example, the heat shrinkable tube 99 is made of a resin material. The heat shrinkable tube 99 is positioned so as to completely cover the partially manufactured heating system 30 that has passed through manufacturing step S16, and shrinks when heated in this state, thereby securing each component laminated onto the outer side of the accommodating portion 50. With such a configuration, it is possible to prevent positional displacement and the like of each component laminated onto the outer side of the accommodating portion 50.

The manufacturing steps of the heating system 30 and the configuration of the heating system 30 have been described above.

### <3. Modified examples>

### (1) First modified example

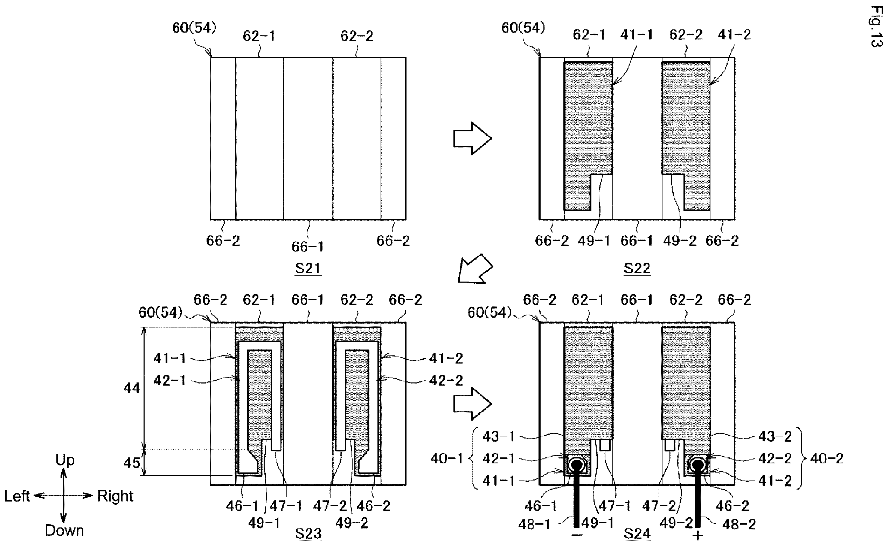

In the above embodiment, an example was described in which the second end portions 47 of the resistive heating layers 42 are connected to the non-pressing portion 66, but the present disclosure is not limited to such an example. The second end portions 47 of the resistive heating layers 42 may be connected to the pressing portion 62. Such a modified example will be described with reference to Figure 13.

Figure 13 is a diagram illustrating an example of the steps for manufacturing the heating system 30 according to the present modified example. The steps for manufacturing the heating system 30 according to the present modified example proceed sequentially through manufacturing steps S21 to S24 illustrated in Figure 13 and then through manufacturing steps S15 to S17 illustrated in Figure 10. That is, the steps for manufacturing the heating system 30 according to the present modified example include manufacturing steps S21 to S24 instead of manufacturing steps S11 to S14 of Figure 9. In the following, points of difference from manufacturing steps S11 to S14 will mainly be described, and descriptions of similar points will be omitted.

Manufacturing step S21 of Figure 13 is the same as manufacturing step S11 of Figure 9.

In manufacturing step S22 of Figure 13, the first electrically insulating layers 41 are laminated onto the pressing portions 62. However, in the present modified example, a cutout 49-1 is provided in a lower portion of the first electrically insulating layer 41-1, exposing a portion of the pressing portion 62-1. Similarly, a cutout 49-2 is provided in a lower portion of the first electrically insulating layer 41-2, exposing a portion of the pressing portion 62-2.

In manufacturing step S23 of Figure 13, the resistive heating layers 42 are laminated onto the outer sides of the first electrically insulating layers 41 laminated onto the pressing portions 62 of the partially manufactured heating system 30 that has passed through manufacturing step S22. However, in the present modified example, the second end portion 47-1 of the resistive heating layer 42-1 protruding from the first electrically insulating layer 41-1 is connected to the pressing portion 62-1 exposed in the cutout 49-1 of the first electrically insulating layer 41-1. Similarly, the second end portion 47-2 of the resistive heating layer 42-2 that protrudes from the first electrically insulating layer 41-2 is connected to the pressing portion 62-2 exposed in the cutout 49-2 of the first electrically insulating layer 41-1. With this configuration, it is possible for the resistive heating layers 42 to be laminated only onto the outer sides of the flat pressing portions 62. The occurrence of defects such as misalignment and peeling of the resistive heating layers 42 can therefore be prevented more effectively than in a case in which the second end portions 47 of the resistive heating layers 42 are connected to the curved non-pressing portions 66.

In manufacturing step S24 of Figure 13, the second electrically insulating layers 43 are laminated onto the outer sides of the first electrically insulating layers 41 and the resistive heating layers 42 laminated onto the pressing portions 62 of the partially manufactured heating system 30 that has passed through manufacturing step S23. However, in the present modified example, a cutout 49-1 is also provided in a lower portion of the second electrically insulating layer 43-1, in the same manner as in the first electrically insulating layer 41-1. Similarly, a cutout 49-2 is also provided in a lower portion of the second electrically insulating layer 43-2, in the same manner as in the first electrically insulating layer 41-2.

Further, in manufacturing step S24, the conducting wire 48-1 is connected to the resistive heating layer 42-1 and the conducting wire 48-2 is connected to the resistive heating layer 42-2.

### (2) Second modified example

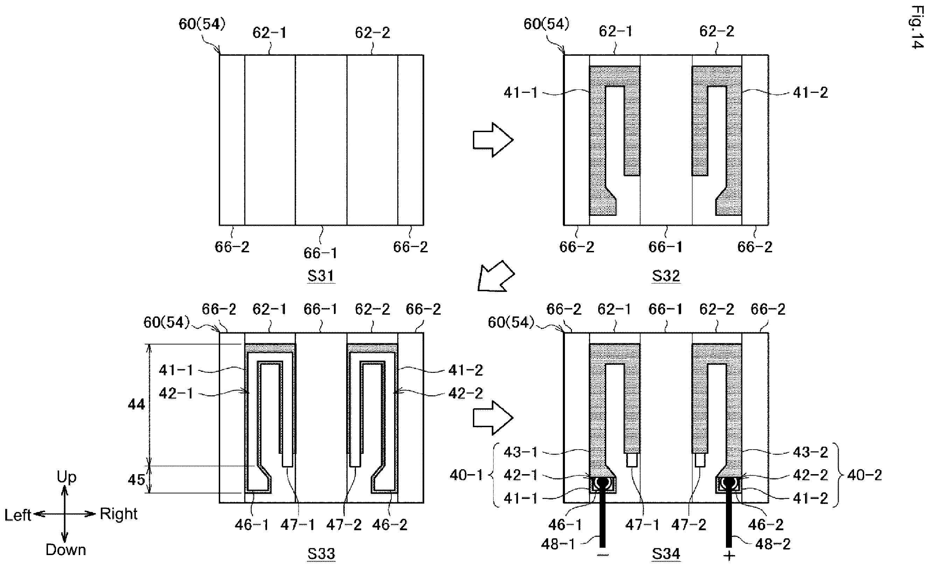

The first electrically insulating layers 41 and the second electrically insulating layers 43 may have any shape, provided that they are shaped to cover the resistive heating layers 42 in such a way as to sandwich the same from both sides. In the following, as a second modified example, another example of the shape that the first electrically insulating layers 41 and the second electrically insulating layers 43 may take is described with reference to Figure 14. In the following, the second modified example is described as a further modified example of the first modified example.

Figure 14 is a diagram illustrating an example of the steps for manufacturing the heating system 30 according to the present modified example. The steps for manufacturing the heating system 30 according to the present modified example proceed sequentially through manufacturing steps S31 to S34 illustrated in Figure 14 and then through manufacturing steps S15 to S17 illustrated in Figure 10. That is, the steps for manufacturing the heating system 30 according to the present modified example include manufacturing steps S31 to S34 instead of manufacturing steps S21 to S24 of Figure 13. In the following, points of difference from manufacturing steps S21 to S24 will mainly be described, and descriptions of similar points will be omitted.

Manufacturing step S31 of Figure 14 is the same as manufacturing step S11 of Figure 9.

In manufacturing step S32 of Figure 14, the first electrically insulating layers 41 are laminated onto the pressing portions 62. However, in the present modified example, the first electrically insulating layer 41-1 has a shape that conforms to the resistive heating layer 42-1 to be laminated later. That is, the first electrically insulating layer 41-1 is laminated onto the pressing portion 62-1 in the shape of a single line that moves back and forth in the up-down direction while leaving a gap in the left-right direction. Similarly, the first electrically insulating layer 41-2 has a shape that conforms to the resistive heating layer 42-2 to be laminated later. That is, the first electrically insulating layer 41-2 is laminated onto the pressing portion 62-2 in the shape of a single line that moves back and forth in the up-down direction while leaving a gap in the left-right direction.

In manufacturing step S33 of Figure 14, in the same manner as in manufacturing step S23 of Figure 13, the resistive heating layers 42 are laminated onto the outer sides of the first electrically insulating layers 41 laminated onto the pressing portions 62 of the partially manufactured heating system 30 that has passed through manufacturing step S32.

In manufacturing step S34 of Figure 14, the second electrically insulating layers 43 are laminated onto the outer sides of the first electrically insulating layers 41 and the resistive heating layers 42 laminated onto the pressing portions 62 of the partially manufactured heating system 30 that has passed through manufacturing step S33. However, in the present modified example, the second electrically insulating layer 43-1 has a similar shape to the first electrically insulating layer 41-1. Similarly, the second electrically insulating layer 43-2 has a similar shape to the first electrically insulating layer 41-2.

Further, in manufacturing step S34, the conducting wire 48-1 is connected to the resistive heating layer 42-1 and the conducting wire 48-2 is connected to the resistive heating layer 42-2.

As described hereinabove, the first electrically insulating layers 41 and the second electrically insulating layers 43 according to the present modified example are in the shape of a single line that moves back and forth in the up-down direction while leaving a gap in the left-right direction. The outer heat diffusion layer 90, to be laminated later, consequently comes into direct contact with the pressing portions 62 exposed in the left-right direction gaps in the first electrically insulating layer 41 and the second electrically insulating layer 43. Consequently, the thermal diffusion effect of the outer heat diffusion layer 90 can also be exhibited with respect to the pressing portions 62, enabling a further improvement in the heating efficiency.

### (3) Third modified example

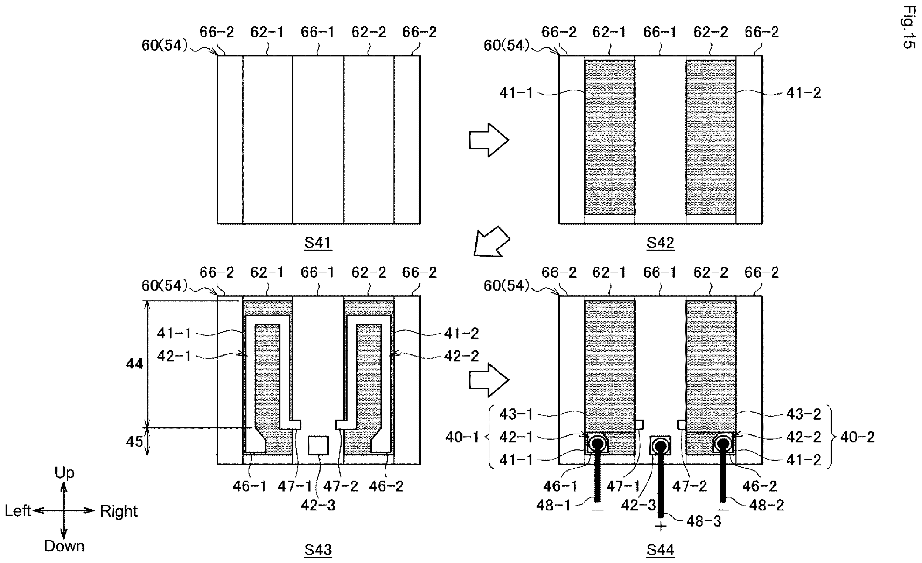

Although an example in which the resistive heating layer 42-1 and the resistive heating layer 42-2 form one series circuit has been described above, the present disclosure is not limited to such an example. The resistive heating layer 42-1 and the resistive heating layer 42-2 may form a parallel circuit. Such a modified example will be described with reference to Figure 15.

Figure 15 is a diagram illustrating an example of the steps for manufacturing the heating system 30 according to the present modified example. The steps for manufacturing the heating system 30 according to the present modified example proceed sequentially through manufacturing steps S41 to S44 illustrated in Figure 15 and then through manufacturing steps S15 to S17 illustrated in Figure 10. That is, the steps for manufacturing the heating system 30 according to the present modified example include manufacturing steps S41 to S44 instead of manufacturing steps S11 to S14 of Figure 9. In the following, points of difference from manufacturing steps S11 to S14 will mainly be described, and descriptions of similar points will be omitted.

Manufacturing step S41 of Figure 15 is the same as manufacturing step S11 of Figure 9.

Manufacturing step S42 of Figure 15 is the same as manufacturing step S12 of Figure 9.

In manufacturing step S43 of Figure 15, in the same manner as in manufacturing step S13 of Figure 9, the resistive heating layers 42-1 and 42-2 are laminated onto the outer sides of the first electrically insulating layers 41-1 and 41-2 laminated onto the pressing portions 62 of the partially manufactured heating system 30 that has passed through manufacturing step S42.

In addition, in the present modified example, in manufacturing step S43 a rectangular resistive heating layer 42-3 is laminated onto a lower portion of the non-pressing portion 66-1. The resistive heating layer 42-3 is laminated in the non-heat generating region 45. That is, the resistive heating layer 42-3 is configured to be wide, similar to the first end portion 46-1 of the resistive heating layer 42-1 and the first end portion 46-2 of the resistive heating layer 42-2. This makes it possible to prevent the generation of heat in the resistive heating layer 42-3 and to prevent the transfer of heat to the conducting wires 48, and also to prevent the connecting parts between the conducting wires 48 and the resistive heating layers 42 from being damaged by heat.

In manufacturing step S44 of Figure 15, the second electrically insulating layers 43 are laminated onto the outer sides of the first electrically insulating layers 41 and the resistive heating layers 42 laminated onto the pressing portions 62 of the partially manufactured heating system 30 that has passed through manufacturing step S43, in the same manner as in manufacturing step S14 of Figure 9.

Further, in manufacturing step S44, the conducting wire 48-1 is connected to the resistive heating layer 42-1 and the conducting wire 48-2 is connected to the resistive heating layer 42-2, in the same manner as in manufacturing step S14 of Figure 9. However, the conducting wire 48-1 and the conducting wire 48-2 are each connected to the negative electrode of the power source unit 111.

In addition, in the present modified example, in manufacturing step 44 a conducting wire 48-3 is connected to the resistive heating layer 42-3. The conducting wire 48-3 is connected to the positive electrode of the power source unit 111. As a result, the conducting wire 48-3 connected to the power source unit 111 is connected to the accommodating portion 50. Then, the second end portion 47-1 of the resistive heating layer 42-1 is electrically connected via the accommodating portion 50 to the conducting wire 48-3 connected to the accommodating portion 50 (more precisely, to the resistive heating layer 42-3). Therefore, the conducting wire 48-1, the resistive heating layer 42-1, the accommodating portion 50, the resistive heating layer 42-3, and the conducting wire 48-3 form a first circuit connected to the power source unit 111. Meanwhile, the second end portion 47-2 of the resistive heating layer 42-2 is electrically connected via the accommodating portion 50 to the conducting wire 48-3 connected to the accommodating portion 50 (more precisely, to the resistive heating layer 42-3). Therefore, the conducting wire 48-2, the resistive heating layer 42-2, the accommodating portion 50, the resistive heating layer 42-3, and the conducting wire 48-3 form a second circuit connected to the power source unit 111. The first circuit and second circuit described above constitute one parallel circuit. When the power source unit 111 supplies electric power to this parallel circuit, heat can be generated in the resistive heating layer 42-1 and the resistive heating layer 42-2.

### (4) Fourth modified example

Although examples in which the resistive heating layers 42 are connected to the power source unit 111 via the accommodating portion 50 have been described above, the present disclosure is not limited to such examples. The resistive heating layers 42 may be connected to the power source unit 111 without passing through the accommodating portion 50. Such a modified example will be described with reference to Figure 16.

Figure 16 is a diagram illustrating an example of the steps for manufacturing the heating system 30 according to the present modified example. The steps for manufacturing the heating system 30 according to the present modified example proceed sequentially through manufacturing steps S51 to S54 illustrated in Figure 16 and then through manufacturing steps S15 to S17 illustrated in Figure 10. That is, the steps for manufacturing the heating system 30 according to the present modified example include manufacturing steps S51 to S54 instead of manufacturing steps S11 to S14 of Figure 9. In the following, points of difference from manufacturing steps S11 to S14 will mainly be described, and descriptions of similar points will be omitted.

Manufacturing step S51 of Figure 16 is the same as manufacturing step S11 of Figure 9.

Manufacturing step S 5 2 of Figure 16 is the same as manufacturing step S 1 2 of Figure 9.

In manufacturing step S53 of Figure 16, the resistive heating layers 42 are laminated onto the outer sides of the first electrically insulating layers 41 laminated onto the pressing portions 62 of the partially manufactured heating system 30 that has passed through manufacturing step S52. However, in the present modified example, both the first end portions 46 and the second end portions 47, which are the two ends of each of the resistive heating layers 42, are disposed within the first electrically insulating layer 41. In particular, the first end portions 46 and the second end portions 47 are disposed on lower end portions of the first electrically insulating layers 41.

In manufacturing step S54 of Figure 16, the second electrically insulating layers 43 are laminated onto the outer sides of the first electrically insulating layers 41 and the resistive heating layers 42 laminated onto the pressing portions 62 of the partially manufactured heating system 30 that has passed through manufacturing step S53, in the same manner as in manufacturing step S14 of Figure 9.

Furthermore, in the present modified example, in manufacturing step S54 the conducting wires 48 connected to the power source unit 111 are connected to each of the first end portions 46 and the second end portions 47 of the resistive heating layers 42. Specifically, the conducting wire 48-1 connected to the positive electrode of the power source unit 111 is connected to the first end portion 46-1 of the resistive heating layer 42-1. A conducting wire 48-4 connected to the negative electrode of the power source unit 111 is connected to the second end portion 47-1 of the resistive heating layer 42-1. Therefore, the conducting wire 48-1, the resistive heating layer 42-1 and the conducting wire 48-4 form a first circuit connected to the power source unit 111. Meanwhile, the conducting wire 48-2 connected to the negative electrode of the power source unit 111 is connected to the first end portion 46-2 of the resistive heating layer 42-2. A conducting wire 48-5 connected to the positive electrode of the power source unit 111 is connected to the second end portion 47-2 of the resistive heating layer 42-2. Therefore, the conducting wire 48-2, the resistive heating layer 42-2 and the conducting wire 48-5 form a second circuit connected to the power source unit 111. The first circuit and second circuit described above constitute one parallel circuit. When the power source unit 111 supplies electric power to this parallel circuit, heat can be generated in the resistive heating layer 42-1 and the resistive heating layer 42-2.

It should be noted that the operations of the first circuit and the second circuit constituting the parallel circuit may be controlled individually or collectively. That is, the first circuit and the second circuit may be supplied with different powers or may be supplied with the same power.

### (5) Fifth modified example

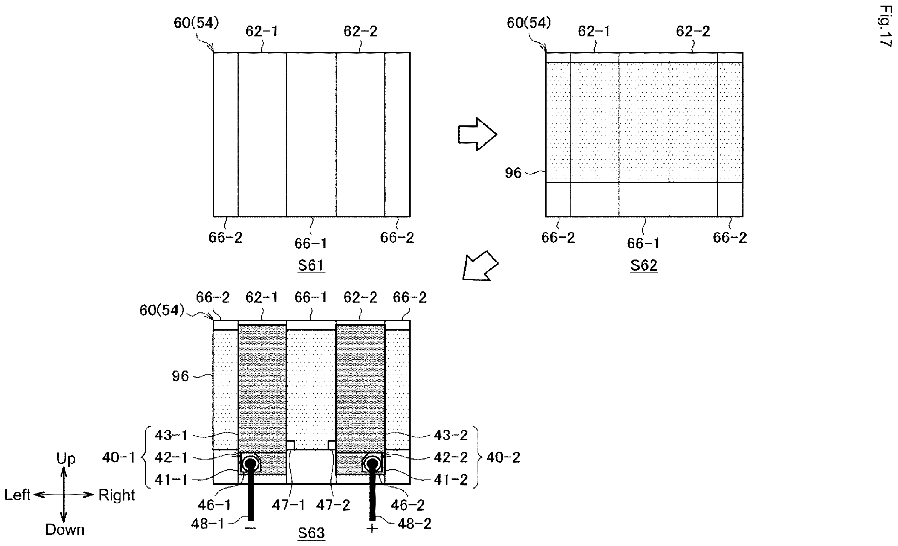

In the above embodiments, examples were described in which the outer heat diffusion layer 90 is laminated onto the outer sides of the heating units 40, but the present disclosure is not limited to such examples. A heat diffusion layer may be laminated on the inner side of the heating units 40. Such a modified example will be described with reference to Figure 17.

Figure 17 is a diagram illustrating an example of the steps for manufacturing the heating system 30 according to the present modified example. The steps for manufacturing the heating system 30 according to the present modified example proceed sequentially through manufacturing steps S61 and S62 illustrated in Figure 17, then manufacturing steps S12 to S14 illustrated in Figure 9, and then manufacturing steps S15 to S17 illustrated in Figure 10. That is, the steps for manufacturing the heating system 30 according to the present modified example include manufacturing steps S61 and S62 instead of manufacturing step S11 of Figure 9. Manufacturing step S65 illustrated in Figure 17 illustrates the state of the partially manufactured heating system 30 that has passed through manufacturing steps S61, S62, and S12 to S14. In the following, points of difference from manufacturing steps S11 to S17 illustrated in Figure 9 and Figure 10 will mainly be described, and descriptions of similar points will be omitted.

Manufacturing step S61 of Figure 17 is the same as manufacturing step S11 of Figure 9.

In manufacturing step S62 of Figure 17, an inner heat diffusion layer 96 is laminated onto the outer side of the side wall 54 of the accommodating portion 50 using a plating process. The inner heat diffusion layer 96 is an example of a first heat diffusion layer that is laminated onto the outer side of the side wall 54 of the accommodating portion 50, inward of the heating units 40, and that diffuses the heat of the heating units 40 inward of the heating units 40. A plating process is a process in which the surface of an object is coated thinly with metal. The inner heat diffusion layer 96 is made of a material that can be plated, and that has a higher thermal conductivity than the material constituting the accommodating portion 50. Furthermore, it is desirable that the inner heat diffusion layer 96 is made of a material that has a higher electrical conductivity than the material constituting the accommodating portion 50. An example of a material that may constitute the inner heat diffusion layer 96 is silver. With this configuration, the heat of the heating units 40, to be laminated onto the pressing portions 62 later, can be diffused throughout the entire accommodating portion 50 including the non-pressing portions 66. As a result, the stick-type substrate 150 accommodated in the accommodating portion 50 can be heated efficiently. It should be noted that the inner heat diffusion layer 96 may be laminated using any means, besides a plating process, such as a thermal spraying process in which metal particles are sprayed to form a coating, or processing in which a paste-like material is applied and fired. In addition, the inner heat diffusion layer 96 may additionally be plated with nickel or gold, for example. This makes it possible to prevent degradation of the inner heat diffusion layer 96, such as oxidation.

Here, it is desirable that the inner heat diffusion layer 96 is laminated so as to overlap the region in which the heat generating regions 44 of the heating units 40 are disposed. With this configuration, the heat from the heating units 40 can be efficiently diffused. Meanwhile, it is desirable that the inner heat diffusion layer 96 is laminated so as to avoid the region in which the non-heat generating regions 45 of the heating units 40 are disposed. With this configuration, it is possible to prevent heat transfer to the conducting wires 48 and to prevent the connecting parts between the conducting wires 48 and the resistive heating layers 42 from being damaged by heat.

Thereafter, manufacturing steps S12 to S14 of Figure 9 are performed to manufacture the partially manufactured heating system 30 illustrated in manufacturing step S63 of Figure 17. That is, the first electrically insulating layers 41, the resistive heating layers 42 and the second electrically insulating layers 43 are each laminated sequentially using a printing process or a vapor deposition process, further outward than the inner heat diffusion layer 96 laminated onto the outer sides of the pressing portions 62. Here, if the electrical conductivity of the inner heat diffusion layer 96 is higher than the electrical conductivity of the accommodating portion 50, it is desirable that the second end portions 47 of the resistive heating layers 42 are connected to the inner heat diffusion layer 96, as illustrated in manufacturing step S63. In this case, the second end portions 47 may be connected to the inner heat diffusion layer 96 on the pressing portions 62 or may be connected to the inner heat diffusion layer 96 on the non-pressing portions 66. This configuration makes it possible to facilitate the conduction of electricity between the resistive heating layer 42-1 and the resistive heating layer 42-2. Of course, the present modified example may be combined with the third modified example, and the conducting wire 48 that is connected to the power source unit 111 may be connected to the accommodating portion 50. In this case, the resistive heating layers 42 are connected to the power source unit 111 via the inner heat diffusion layer 96 and the accommodating portion 50.

Alternatively, the second end portions 47 of the resistive heating layers 42 may be connected to the accommodating portion 50 that is exposed from the inner heat diffusion layer 96, avoiding the inner heat diffusion layer 96. For example, the accommodating portion 50 and the resistive heating layers 42 may be made of the same SUS and electrically connected by welding. With this configuration, it is possible to prevent a decrease in durability resulting from intermetallic corrosion or solid solution.

### (6) Sixth modified example

Although examples in which the accommodation portion 50 is a tubular body in the shape of a circular cylinder have been described above, the present disclosure is not limited to such examples. The accommodating portion 50 may have any shape, provided that the shape has a pressing portion 62 that is a flat plate. Such a modified example will be described with reference to Figure 18.

Figure 18 is a diagram illustrating schematically an example of the configuration of the accommodating portion 50 and the stick-type substrate 150 according to the present modified example. As illustrated in Figure 18, the accommodating portion 50 may be a bottomed rectangular tube in which the shape of the surfaces perpendicular to the up-down direction are rectangular. In the present modified example, not only the pressing portions 62, but also the non-pressing portions 66 are configured as flat plates. That is, the accommodating portion 50 according to the present modified example is configured by connecting the bottom wall 56 to the lower end of the side wall 54, which is configured by alternately connecting the pair of pressing portions 62, which are flat plates, and the pair of non-pressing portions 66, which are flat plates. However, it is desirable that the length of the non-pressing portions 66 in the peripheral direction of the accommodating portion 50 is configured to be less than the length of the pressing portions 62. That is, it is desirable that the accommodating portion 50 is configured such that the shape thereof in a plane perpendicular to the up-down direction is rectangular, with the pressing portions 62 constituting the long sides and the non-pressing portions 66 constituting the short sides. Furthermore, it desirable that the heating units 40 are disposed on the pressing portions 62.

As illustrated in Figure 18, the stick-type substrate 150 may be configured in a prismatic shape having a rectangular cross-sectional shape to match the shape of the accommodating portion 50. For example, the stick-type substrate 150 may be configured in the form of a thin card.

With this configuration, the thinly configured stick-type substrate 150 can be heated while being sandwiched between the heating units 40, and thus the temperature can easily be increased up to the central portion of the stick-type substrate 150.

### (7) Seventh modified example

Although examples in which the resistive heating layers 42 protrude from the first electrically insulating layers 41 in a direction along the outer peripheral surface of the accommodating portion 50 have been described above, the present disclosure is not limited to such examples. For example, the resistive heating layers 42 may protrude from the first electrically insulating layers 41 in a direction perpendicular to the outer peripheral surface of the accommodating portion 50. Such a modified example will be described with reference to Figure 19.

Figure 19 is a diagram illustrating an example of the steps for manufacturing the heating system 30 according to the present modified example. The steps for manufacturing the heating system 30 according to the present modified example proceed sequentially through manufacturing steps S71 to S74 illustrated in Figure 19 and then through manufacturing steps S15 to S17 illustrated in Figure 10. That is, the steps for manufacturing the heating system 30 according to the present modified example include manufacturing steps S71 to S74 instead of manufacturing steps S11 to S14 of Figure 9. In the following, points of difference from manufacturing steps S11 to S14 will mainly be described, and descriptions of similar points will be omitted. Furthermore, although the manufacturing steps relating to one of the two heating units 40 will mainly be described below, the other heating unit 40 may also be manufactured using the same manufacturing steps.

In manufacturing step S71 of Figure 19, the first electrically insulating layer 41 is subjected to via machining to form a through-hole 41a. The first electrically insulating layer 41 according to the present modified example may be a ceramic substrate prior to sintering, such as a green sheet. The through-hole 41a in the first electrically insulating layer 41 is then filled with an electrically conductive material 42a. The electrically conductive material 42a is made of any material that is electrically conductive. The material of the conductive material 42a may be the same as the material of the resistive heating layers 42.

In manufacturing step S72 of Figure 19, the resistive heating layer 42 is laminated onto the first electrically insulating layer 41 that has passed through manufacturing step S71. Here, the second end portion 47 of the resistive heating layer 42 is disposed on the through-hole 41a. The second end portion 47 of the resistive heating layer 42 is then connected to the conductive material 42a with which the through-hole 41a has been filled.

In manufacturing step S73 of Figure 19, the second electrically insulating layer 43 is laminated onto the first electrically insulating layer 41 and the resistive heating layer 42 that have passed through manufacturing step S72. For example, the second electrically insulating layer 43 is bonded to the first electrically insulating layer 41 so as to sandwich the resistive heating layer 42 in a state in which the first end portion 46 of the resistive heating layer 42 is exposed. The second electrically insulating layer 43 according to the present modified example may be a ceramic substrate prior to sintering, such as a green sheet.

The heating unit 40 according to the present modified example is manufactured by means of the manufacturing steps described above.

In manufacturing step S74 of Figure 19, the heating unit 40 that has passed through manufacturing step S73 is laminated onto the outer side of the pressing portion 62 of the accommodating portion 50. For example, the heating unit 40 is affixed to the outer side of the pressing portion 62 of the accommodating portion 50 and then fired. As a result, the second end portion 47 of the resistive heating layer 42 is connected to the accommodating portion 50 via the electrically conductive material 42a disposed in the through-hole 41a. Meanwhile, the conducting wire 48 is connected to the first end portion 46 of the resistive heating layer 42.

The steps for manufacturing the heating system 30 according to the present modified example have been described above.

According to the present modified example, in the same manner as in the above embodiments, the resistive heating layers 42 are electrically connected to the power source unit 111 via the accommodating portion 50. The conductive material 42a disposed in the through-hole 41a can also be regarded as a portion of the resistive heating layer 42. That is, the resistive heating layer 42 may protrude from the first electrically insulating layer 41 in a direction penetrating through the first electrically insulating layer 41, and be connected to the accommodating portion 50. The through-hole 41a in the present modified example corresponds to the cutout 49 in the above embodiment, in that it is configured to expose the pressing portion 62 formed in the first electrically insulating layer 41.

It should be noted that, although an example in which the heating unit 40 is manufactured separately and is then affixed to the outer side of the accommodating portion 50 has been described using Figure 19, the present disclosure is not limited to such an example. In the same manner as in the above embodiments, the first electrically insulating layers 41, the resistive heating layers 42, and the second electrically insulating layers 43 may be laminated sequentially onto the accommodating portion 50.

### <4. Supplementary information>

Although preferred embodiments of the present disclosure have been described in detail above with reference to the accompanying drawings, the present disclosure is not limited to such examples. It is obvious that a person having an ordinary level of knowledge in the technical field to which the present disclosure belongs could conceive of various modified examples or variations within the scope of the technical concepts set forth in the claims, and these modified examples and variations will naturally be understood to fall within the technical scope of the present disclosure.

Various methods are conceivable for manufacturing the accommodating portion 50 in the form of a tubular body. As an example, the accommodating portion 50 in the form of a tubular body may be formed by subjecting a sheet material to a drawing process. As another example, the accommodating portion 50 in the form of a tubular body may be formed by bending a sheet material and welding the joints. In the latter case, the heating units 40 may be laminated onto the sheet material. Then, the accommodating portion 50 with the heating units 40 laminated thereon may be formed by bending the sheet material with the heating units 40 laminated thereon and welding the joints.

Although examples in which the holding portion 60 has two pressing portions 62 and two non-pressing portions 66 have been described above, the present disclosure is not limited to such examples. For example, the holding portion 60 may have three or more pressing portions 62 and three or more non-pressing portions 66.

Although examples in which each of the first electrically insulating layers 41, the resistive heating layers 42 and the second electrically insulating layers 43 constituting the heating units 40 are laminated using a printing process or a vapor deposition process have been described above, the present disclosure is not limited to such examples. As an example, the first electrically insulating layers 41 and the second electrically insulating layers 43 may be laminated by applying or transferring a paste-like material. As another example, the resistive heating layers 42 may comprise a metal foil processed into a predetermined shape, and may be placed on the first electrically insulating layers 41. If the resistive heating layers 42 comprise a metal foil, the metal foil may be placed on a carrier tape, and the first electrically insulating layers 41 may be printed thereon, and then the resulting printed material may be collectively transferred to the accommodating portion 50. If the resistive heating layers 42 comprise a metal foil, the resistive heating layers 42 and the accommodating portion 50 may be electrically connected by welding. Alternatively, for example, the heating unit 40 may be manufactured separately and affixed to the outer side of the accommodating portion 50.

Although examples in which the connecting parts between the resistive heating layers 42 and the conducting wires 48 are exposed without being covered by the second electrically insulating layers 43 have been described above, the present disclosure is not limited to such examples. The connecting parts between the resistive heating layers 42 and the conducting wires 48 may be covered by the second electrically insulating layers 43.

Although examples in which the resistive heating layers 42 and the conducting wires 48 are connected directly have been described above, the present disclosure is not limited to such examples. The resistive heating layers 42 and the conducting wires 48 may be connected indirectly. As an example, the conducting wires 48 may be connected to the resistive heating layers 42 by way of an electrically conductive leaf spring. As another example, the conducting wires 48 may be connected to the resistive heating layers 42 by way of pogo pins. The inhalation device 100 may be manufactured by assembling a plurality of components, including the heating system 30, and during the assembly process, the heating system 30 may be fitted into a main body that includes the power source unit 111 and the like. At this time, the lower portion of the heating system 30 may be fitted into a socket provided in the main body, and the plate spring or pogo pin described above may be provided in the socket. In this case, since the resistive heating layers 42 and the power source unit 111 can be electrically connected when the lower portion of the heating system 30 is fitted into the socket, the steps for manufacturing the inhalation device 100 can be simplified. It should be noted that if the resistive heating layers 42 and the conducting wires 48 are connected indirectly, it is desirable that the resistive heating layers 42 in their entirety, or at least the first end portions 46 thereof, which are the points of contact with the conducting wires 48, are plated with nickel, gold, or the like. This configuration allows for a stronger electrical connection between the resistive heating layers 42 and the plate springs or the pogo pins. It should be noted that the accommodating portion 50 and the conducting wires 48 may similarly be directly connected or indirectly connected.

Although examples in which the contact points (i.e., the first end portions 46) between the resistive heating layers 42 and the conducting wires 48 are located on the pressing portions 62 have been described above, the present disclosure is not limited to such examples. For example, the first electrically insulating layers 41 and the resistive heating layers 42 may be extended to the bottom wall 56 of the accommodating portion 50, and the conducting wires 48 may be directly or indirectly connected to the resistive heating layers 42 on the bottom wall 56 of the accommodating portion 50.

Although examples in which the outer heat diffusion layer 90 covers the holding portion 60 have been described above, the outer heat diffusion layer 90 may cover not only the holding portion 60 but also the non-holding portion 69. Similarly, although examples in which the heat insulating sheet 71 covers the holding portion 60 have been described above, the heat insulating sheet 71 may cover not only the holding portion 60 but also the non-holding portion 69.

Although an example in which the vertically long PI tape 92 is bonded to the non-pressing portions 66 when the outer heat diffusion layer 90 is laminated to the accommodating portion 50 has been described above, the present disclosure is not limited to such an example. The vertically long PI tape 92 may be bonded to the second electrically insulating layers 43 laminated onto the pressing portions 62.

Although examples in which the stick-type substrate 150 includes the substrate portion 151 and the mouthpiece portion 152 have been described above, the present disclosure is not limited to such examples. The stick-type substrate 150 may include only the substrate portion 151. Then, the inhalation device 100 may include the mouthpiece portion 152.

For example, the mouthpiece portion 152 may be removably attached to the opening 52 of the accommodating portion 50.

Two or more of the above embodiments and modified examples may be combined, as appropriate. As an example, the above embodiments may be combined with the fifth modified example. That is, the heating system 30 may include both the outer heat diffusion layer 90 and the inner heat diffusion layer 96. As another example, the accommodating portion 50 may include four or more pressing portions 62, and any two types of heating unit 40 among the heating units 40 illustrated in Figures 9 and Figure 13 to Figure 17 may be disposed on one accommodating portion 50. As another example, any one type of heating unit 40 among the heating units 40 illustrated in Figures 9 and Figure 13 to Figure 17 may be disposed on the accommodating portion 50 illustrated in Figure 18.

Although examples in which the conducting wires 48 are connected to at least one of the two ends of each resistive heating layer 42 have been described above, the present disclosure is not limited to such examples. As an example, the accommodating portion 50 may have three or more pressing portions 62, and both ends of the resistive heating layer 42 disposed on the pressing portion 62 located at the center of the three pressing portions 62 may be connected to the accommodating portion 50. Then, resistive heating layers 42 having one end connected to the power source unit 111 may be disposed on each of the two pressing portions 62 adjacent to and on both sides thereof, and the three resistive heating layers 42 may constitute one series circuit. As another example, the accommodating portion 50 may include two pressing portions 62, resistive heating layers 42 having both ends connected to the accommodating portion 50 may be disposed on each of the two pressing portions 62, and conducting wires connected to the power source unit 111 may be connected to each of the two non-pressing portions 66. In this case, the two resistive heating layers 42 form a parallel circuit.

It should be noted that configurations such as the following also fall within the technical scope of the present disclosure.
(1) An aerosol generation system comprising a tubular body that accommodates a substrate containing an aerosol source,
   a plurality of resistive heating layers that are laminated onto the outer side of a side wall of the tubular body, and
   a first heat diffusion layer that is laminated onto the outer side of the side wall of the tubular body, inward of the resistive heating layers.
(2) The aerosol generation system as set forth in (1), wherein: the side wall of the tubular body includes a plurality of first side walls having a planar outer surface and a plurality of second side walls different from the first side walls;
   the first side walls and the second side walls are arranged alternately along the circumferential direction of the tubular body; and
   two of the resistive heating layers are laminated using a vapor deposition process or a printing process onto the outer sides of two of the first side walls that are adjacent to and on both sides of the second side walls, in a state in which the resistive heating layers are spaced apart at the second side wall.s
(3) The aerosol generation system as set forth in (2), further comprising a plurality of first electrically insulating layers that are laminated using a vapor deposition process or a printing process onto the outer sides of the first side walls, inward of the resistive heating layers, wherein
   the tubular body is made of an electrically conductive material.
(4) The aerosol generation system as set forth in (3), wherein the part of the outer periphery of the tubular body on which the first electrically insulating layers are laminated occupies less than 50% of the outer periphery of the tubular body.
(5) The aerosol generation system as set forth in (3) or (4), wherein the first electrically insulating layers have a shape that conforms to the resistive heating layers.
(6) The aerosol generation system as set forth in any one of (3) to (5), further comprising a plurality of second electrically insulating layers that are laminated outward of the resistive heating layers using a vapor deposition process or a printing process, wherein
   at least portions of the resistive heating layers are sandwiched between the first electrically insulating layers and the second insulating layers.
(7) The aerosol generation system as set forth in any one of (3) to (6), further comprising a power source unit for supplying power to the resistive heating layers, wherein
   the first heat diffusion layer is made of a material having a higher electrical conductivity than the tubular body;
   the first electrically insulating layers are laminated outward of the first heat diffusion layer; and
   at least one of the two end portions of each resistive heating layer protrudes from the first electrically insulating layer and is connected to the first heat diffusion layer, and is electrically connected to the power source unit via the first heat diffusion layer.
(8) The aerosol generation system as set forth in (7), wherein at least one of the two end portions of each resistive heating layer protrudes from the first electrically insulating layer and is connected to the first heat diffusion layer, and is electrically connected via the first heat diffusion layer to another resistive heating layer adjacent to the resistive heating layer.
(9) The aerosol generation system as set forth in (7) or (8), wherein a conducting wire that is connected to the power source unit is connected to the tubular body, and
   one of the two end portions of each resistive heating layer protrudes from the first electrically insulating layer and is connected to the first heat diffusion layer, and is electrically connected via the first heat diffusion layer and the tubular body to the conducting wire that is connected to the tubular body.
(10) The aerosol generation system as set forth in any one of (7) to (9), wherein the end portion that protrudes from each first electrically insulating layer, among the two end portions of each resistive heating layer, is connected to the first heat diffusion layer on the first side wall.
(11) The aerosol generation system as set forth in any one of (7) to (9), wherein the end portion that protrudes from each first electrically insulating layer, among the two end portions of each resistive heating layer, protrudes from the first side wall and is connected to the first heat diffusion layer on the second side wall.
(12) The aerosol generation system as set forth in any one of (7) to (11), wherein a conducting wire that is connected to the power source unit is connected to one of the two end portions of each resistive heating layer.
(13) The aerosol generation system as set forth in any one of (1) to (12), further comprising a power source unit for supplying power to the resistive heating layers, wherein
   a conducting wire that is connected to the power source unit is connected to each of the two end portions of each resistive heating layer.
(14) The aerosol generation system as set forth in (12) or (13), wherein, among the two end portions of each resistive heating layer, the end portion to which the conducting wire that is connected to the power source unit is connected is configured to be wider than other parts thereof.
(15) The aerosol generation system as set forth in any one of (1) to (14), further comprising a second heat diffusion layer that is wrapped and laminated onto the outer side of the side wall of the tubular body, outward of the resistive heating layers.
(16) The aerosol generation system as set forth in any one of (1) to (15), further comprising a heat insulating layer that is wrapped and laminated onto the outer side of the side wall of the tubular body, outward of the resistive heating layers.
(17) The aerosol generation system as set forth in any one of (1) to (12), further comprising a power source unit for supplying power to the resistive heating layers, wherein
   at least one of the two end portions of each resistive heating layer protrudes from the first electrically insulating layer and is connected to the tubular body, and is electrically connected to the power source unit via the tubular body.
(18) The aerosol generation system as set forth in any one of (3) to (17) that cites (2), wherein: the first side walls are flat plates;
   the second side walls are curved plates that are curved to the outside of the tubular body along the circumferential direction of the tubular body; and
   the substrate accommodated in the tubular body is pressed by the first side walls.
(19) The aerosol generation system as set forth in any one of (3) to (17) that cites (2), wherein: the first side walls are flat plates;
   the second side walls are flat plates;
   the length of the first side walls in the circumferential direction of the tubular body is greater than the length of the second side walls; and
   the substrate accommodated in the tubular body is pressed by the first side walls.
(20) The aerosol generation system as set forth in any one of (1) to (19), further comprising the substrate.

### [REFERENCE SIGNS LIST]

- 100: inhalation device
- 111: power source unit
- 112: sensor unit
- 113: notification unit
- 114: memory unit
- 115: communication unit
- 116: control unit
- 150: stick-type substrate
- 151: substrate portion
- 152: mouthpiece portion
- 30: heating system
- 40: heating unit
- 41: first electrically insulating layer
- 42: resistive heating layer
- 43: second electrically insulating layer
- 44: heat generating region
- 45: heat generating region
- 46: first end portion
- 47: second end portion
- 48: conducting wire
- 49: cutout
- 50: accommodating portion
- 52: opening
- 54: side wall (54a: inner surface, 54b: outer surface)
- 56: bottom wall (56a: inner surface, 56b: outer surface)
- 58: first guide portion (58a: tapered surface)
- 60: holding portion
- 62: pressing portion (62a: inner surface, 62b: outer surface)
- 66: non-pressing portion (66a: inner surface, 66b: outer surface)
- 67: gap
- 68: boundary
- 69: non-holding portion
- 70: heat insulating portion
- 71: heat insulating sheet
- 72: PI tape
- 73: sealing member
- 80: internal space
- 90: outer heat diffusion layer
- 91: graphite sheet
- 92: vertically long PI tape
- 93: horizontally long PI tape (94: protruding part, 95: protruding part)
- 96: inner heat diffusion layer
- 99: heat shrinkable tube

## Claims

1. An aerosol generation system comprising a tubular body that accommodates a substrate containing an aerosol source,
a plurality of resistive heating layers that are laminated onto the outer side of a side wall of the tubular body, and
a first heat diffusion layer that is laminated onto the outer side of the side wall of the tubular body, inward of the resistive heating layers.

2. The aerosol generation system as claimed in claim 1, wherein: the side wall of the tubular body includes a plurality of first side walls having a planar outer surface and a plurality of second side walls different from the first side walls;
the first side walls and the second side walls are arranged alternately along the circumferential direction of the tubular body; and
two of the resistive heating layers are laminated using a vapor deposition process or a printing process onto the outer sides of two of the first side walls that are adjacent to and on both sides of the second side walls, in a state in which the resistive heating layers are spaced apart at the second side walls.

3. The aerosol generation system as claimed in claim 2, further comprising a plurality of first electrically insulating layers that are laminated using a vapor deposition process or a printing process onto the outer sides of the first side walls, inward of the resistive heating layers, wherein
the tubular body is made of an electrically conductive material.

4. The aerosol generation system as claimed in claim 3, wherein the part of the outer periphery of the tubular body on which the first electrically insulating layers are laminated occupies less than 50% of the outer periphery of the tubular body.

5. The aerosol generation system as claimed in claim 3 or 4, wherein the first electrically insulating layers have a shape that conforms to the resistive heating layers.

6. The aerosol generation system as claimed in any one of claims 3 to 5, further comprising a plurality of second electrically insulating layers that are laminated outward of the resistive heating layers using a vapor deposition process or a printing process, wherein
at least portions of the resistive heating layers are sandwiched between the first electrically insulating layers and the second insulating layers.

7. The aerosol generation system as claimed in any one of claims 3 to 6, further comprising a power source unit for supplying power to the resistive heating layers, wherein
the first heat diffusion layer is made of a material having a higher electrical conductivity than the tubular body;
the first electrically insulating layers are laminated outward of the first heat diffusion layer; and
at least one of the two end portions of each resistive heating layer protrudes from the first electrically insulating layer and is connected to the first heat diffusion layer, and is electrically connected to the power source unit via the first heat diffusion layer.

8. The aerosol generation system as claimed in claim 7, wherein at least one of the two end portions of each resistive heating layer protrudes from the first electrically insulating layer and is connected to the first heat diffusion layer, and is electrically connected via the first heat diffusion layer to another resistive heating layer adjacent to the resistive heating layer.

9. The aerosol generation system as claimed in claim 7 or 8, wherein a conducting wire that is connected to the power source unit is connected to the tubular body, and
one of the two end portions of each resistive heating layer protrudes from the first electrically insulating layer and is connected to the first heat diffusion layer, and is electrically connected via the first heat diffusion layer and the tubular body to the conducting wire that is connected to the tubular body.

10. The aerosol generation system as claimed in any one of claims 7 to 9, wherein the end portion that protrudes from each first electrically insulating layer, among the two end portions of each resistive heating layer, is connected to the first heat diffusion layer on the first side wall.

11. The aerosol generation system as claimed in any one of claims 7 to 9, wherein the end portion that protrudes from each first electrically insulating layer, among the two end portions of each resistive heating layer, protrudes from the first side wall and is connected to the first heat diffusion layer on the second side wall.

12. The aerosol generation system as claimed in any one of claims 7 to 11, wherein a conducting wire that is connected to the power source unit is connected to one of the two end portions of each resistive heating layer.

13. The aerosol generation system as claimed in any one of claims 1 to 12, further comprising a power source unit for supplying power to the resistive heating layers, wherein
a conducting wire that is connected to the power source unit is connected to each of the two end portions of each resistive heating layer.

14. The aerosol generation system as claimed in claim 12 or 13, wherein, among the two end portions of each resistive heating layer, the end portion to which the conducting wire that is connected to the power source unit is connected is configured to be wider than other parts thereof.

15. The aerosol generation system as claimed in any one of claims 1 to 14, further comprising a second heat diffusion layer that is wrapped and laminated onto the outer side of the side wall of the tubular body, outward of the resistive heating layers.

16. The aerosol generation system as claimed in any one of claims 1 to 15, further comprising a heat insulating layer that is wrapped and laminated onto the outer side of the side wall of the tubular body, outward of the resistive heating layers.

17. The aerosol generation system as claimed in any one of claims 1 to 12, further comprising a power source unit for supplying power to the resistive heating layers, wherein
at least one of the two end portions of each resistive heating layer protrudes from the first electrically insulating layer and is connected to the tubular body, and is electrically connected to the power source unit via the tubular body.

18. The aerosol generation system as claimed in any one of claims 3 to 17 that cites claim 2, wherein: the first side walls are flat plates;
the second side walls are curved plates that are curved to the outside of the tubular body along the circumferential direction of the tubular body; and
the substrate accommodated in the tubular body is pressed by the first side walls.

19. The aerosol generation system as claimed in any one of claims 3 to 17 that cites claim 2, wherein: the first side walls are flat plates;
the second side walls are flat plates;
the length of the first side walls in the circumferential direction of the tubular body is greater than the length of the second side walls; and
the substrate accommodated in the tubular body is pressed by the first side walls.

20. The aerosol generation system as claimed in any one of claims 1 to 19, further comprising the substrate.
